(19) Europäisches Patentamt
European Patent Office
Office européen des brevets

(11) **EP 4 188 192 B1**

(12) **EUROPEAN PATENT SPECIFICATION**

(45) Date of publication and mention
of the grant of the patent:
**08.07.2026 Bulletin 2026/28**

(21) Application number: **21752204.4**

(22) Date of filing: **29.07.2021**

(51) International Patent Classification (IPC):
*A61B 5/00* (2006.01)     *A61B 5/08* (2006.01)
*A61B 5/11* (2006.01)     *A61B 5/087* (2006.01)
*A61B 5/0205* (2006.01)     *G16H 20/40* (2018.01)

(52) Cooperative Patent Classification (CPC):
**A61B 5/4812; A61B 5/0205; A61B 5/0803;
A61B 5/087; A61B 5/1118; A61B 5/4836;
G16H 20/40; G16H 40/67;** A61B 5/0077;
A61B 5/082; A61B 5/0823; A61B 5/4818

(86) International application number:
**PCT/IB2021/056943**

(87) International publication number:
**WO 2022/024046 (03.02.2022 Gazette 2022/05)**

(54) **SYSTEMS FOR DETERMINING MOVEMENT DURING RESPIRATORY THERAPY**

SYSTEME ZUR BESTIMMUNG DER BEWEGUNG WÄHREND EINER ATEMTHERAPIE

SYSTÈMES DE DÉTERMINATION DE MOUVEMENT PENDANT UNE THÉRAPIE RESPIRATOIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.07.2020 US 202062706104 P**

(43) Date of publication of application:
**07.06.2023 Bulletin 2023/23**

(73) Proprietor: **ResMed Sensor Technologies Limited
Sandyford D18 T6T7
Dublin (IE)**

(72) Inventors:
• **RICE, Anna
Dublin, D18 T6T7 (IE)**
• **LYON, Graeme Alexander
Dublin, D18 T6T7 (IE)**
• **MCMAHON, Stephen
Dublin, D18 T6T7 (IE)**
• **SHOULDICE, Redmond
Dublin, D18 T6T7 (IE)**

(74) Representative: **Mathys & Squire
Theatinerstraße 8
80333 München (DE)**

(56) References cited:
WO-A1-2010/091462     WO-A1-2018/050913
WO-A2-2020/092701     US-A1- 2012 302 926
US-A1- 2013 245 502

**Description**

**CROSS-REFERENCE TO RELATED APPLICATIONS**

This application claims the benefit of, and priority to, U.S. Provisional Patent Application No. 62/706,104 filed on July 31, 2020.

**TECHNICAL FIELD**

The present disclosure relates generally to systems and methods for movement and body position detection, and more particularly, to systems and methods for movement and body position detection of a user during respiratory therapy.

**BACKGROUND**

Many individuals suffer from sleep-related respiratory disorders, such as, for example, Periodic Limb Movement Disorder (PLMD), Restless Leg Syndrome (RLS), Sleep-Disordered Breathing (SDB) such as Obstructive Sleep Apnea (OSA), Central Sleep Apnea (CSA), other types of apneas such as mixed apneas and hypopneas, Respiratory Effort Related Arousal (RERA), Cheyne-Stokes Respiration (CSR), respiratory insufficiency, Obesity Hyperventilation Syndrome (OHS), Chronic Obstructive Pulmonary Disease (COPD), Neuromuscular Disease (NMD), rapid eye movement (REM) behavior disorder (also referred to as RBD), dream enactment behavior (DEB), hypertension, diabetes, stroke, insomnia, and chest wall disorders. These disorders are often treated using a respiratory therapy system.
However, some users find such systems to be uncomfortable, difficult to use, expensive, aesthetically unappealing and/or fail to perceive the benefits associated with using the system. As a result, some users will elect not to begin using the respiratory therapy system or discontinue use of the respiratory therapy system absent a demonstration of the severity of their symptoms when respiratory therapy treatment is not used. As a further result, some users will discontinue use of the respiratory therapy system absent encouragement or affirmation that the respiratory therapy system is improving their sleep quality and reducing the symptoms of these disorders. The present disclosure is directed to solving these and other problems. For example, when a user is undergoing respiratory therapy, movement information associated with the user can be used for sleep-wake determination. Thus, a need exists for systems and methods for determining movement associated with users undergoing respiratory therapy.
WO 2020/092701 A2 discloses medical devices used by users with respiratory disorders. The patient treatment devices include a controller, a memory, a sensor, wherein the sensor is configured to collect data from a patient, and the collected data is stored in the memory, the collected data including low resolution data and high resolution data, and a transmitter, wherein the transmitter is controlled by the controller to send either low or high resolution data to an external system, the high resolution data being transmitted based on the occurrence of an event detected based on the low resolution data.

SUMMARY

The invention relates to a system for determining movement of a user while using a respiratory therapy system according to claim 1. Further embodiments are defined by dependent claims 2-15.
Also disclosed herein, but not claimed, is a method for determining movement of a user while using a respiratory therapy system. Flow rate data associated with a plurality of flow rate values of pressurized air directed to an airway of the user of the respiratory therapy system is received. The received flow rate data is analyzed. Based at least in part on the analyzed flow rate data, a movement event associated with the user is determined.
Also disclosed herein, but not claimed, is a method for determining a body position of a user during a respiratory therapy session. Flow rate data associated with the user of a respiratory therapy system is received. The received flow rate data is analyzed to determine one or more features. Based at least in part on the determined one or more features, the body position of the user is determined.
Also disclosed herein, but not claimed, is another method for determining a body position of a user during a respiratory therapy session. Acoustic data associated with the user of a respiratory therapy system is received. The received acoustic data is analyzed to determine one or more features. Based at least in part on the determined one or more features, the body position of the user is determined.
Also disclosed herein, but not claimed, is a method for monitoring a patient while using a respiratory therapy system. A movement event associated with the patient is determined using any of the methods disclosed above, and further described herein. A notification is sent to a monitoring device or personnel. The notification is associated with the determined movement event associated with the patient.
Also disclosed herein, but not claimed, is a method for monitoring a patient while using a respiratory therapy system disclosed. A body position of the patient is determined using the method any of the methods disclosed above, and further described herein. A notification is sent to a monitoring device or personnel. The notification is associated with the determined body position of the patient.

The claimed system includes a control system having one or more processors, and a memory having stored thereon machine readable instructions. The control system is coupled to the memory. Any of the methods disclosed above, and further described herein, is implemented when the machine executable instructions in the memory are executed by at least one of the one or more processors of the control system.

The claimed system for determining movement of a user while using a respiratory therapy system includes a control system configured to implement any of the methods disclosed above, and further described herein.

Also disclosed herein, but not claimed, is a system for determining a body position of while using a respiratory therapy system including a control system configured to implement any of the methods disclosed above, and further described herein.

Also disclosed herein, but not claimed, is a system for monitoring a patient while using a respiratory therapy system including a control system configured to implement any of the methods disclosed above and further described herein.

Also disclosed herein, but not claimed, is a computer program product including instructions which, when executed by a computer, cause the computer to carry out any of the methods disclosed above, and further described herein. In some implementations, the computer program product is a non-transitory computer readable medium.

## BRIEF DESCRIPTION OF THE DRAWINGS

The foregoing and other advantages of the present disclosure will become apparent upon reading the following detailed description and upon reference to the drawings.

FIG. 1 is a functional block diagram of a system, according to some implementations of the present disclosure.
FIG. 2 is a perspective view of at least a portion of the system of FIG. 1, a user wearing a full face mask, and a bed partner, according to some implementations of the present disclosure.
FIG. 3 illustrates the generation of acoustic data in response to an acoustic reflection associated with a user while using a respiratory therapy system, according to some implementations of the present disclosure.
FIG. 4A illustrates flow rate data associated with a user of a respiratory therapy system, according to some implementations of the present disclosure.
FIG. 4B illustrates pressure data associated with a user of a continuous positive airway pressure system, according to some implementations of the present disclosure.
FIG. 4C illustrates pressure data associated with a user of a respiratory therapy system with an expiratory pressure relief module, according to some implementations of the present disclosure.
FIG. 5 is a flow diagram for a method for determining movement of a user while using a respiratory therapy system, according to some implementations of the present disclosure.
FIG. 6A illustrates radio frequency (RF) data measured during a therapy session, according to some implementations of the present disclosure.
FIG. 6B illustrates audio data measured during the therapy session of FIG, 6A, according to some implementations of the present disclosure.
FIG. 6C illustrates processed audio data calculated using the audio data of FIG. 6B, according to some implementations of the present disclosure.
FIG. 6D illustrates movement activity data calculated using the RF data of FIG. 6A and separately using the processed audio data of FIG. 6C, according to some implementations of the present disclosure.
FIG. 7A illustrates EMFIT activity data measured during a first therapy session, according to some implementations of the present disclosure.
FIG. 7B illustrates processed audio data derived from audio signals generated during the first therapy session of FIG. 7A, according to some implementations of the present disclosure.
FIG. 7C illustrates EMFIT activity data measured during a second therapy session, according to some implementations of the present disclosure.
FIG. 7D illustrates audio data measured during the second therapy session of FIG. 7C, according to some implementations of the present disclosure.
FIG. 8 is a flow diagram for a method for determining a body position of a user during a respiratory therapy session, according to some implementations of the present disclosure.
FIG. 9 illustrates a real-time flow signal and a real-time pressure signal measured during a therapy session, according to some implementations of the present disclosure.
FIG. 10 is a flow diagram for a method for determining a body position of a user during a respiratory therapy session, according to some implementations of the present disclosure.

While the present disclosure is susceptible to various modifications and alternative forms, specific implementations and embodiments thereof have been shown by way of example in the drawings and will herein be described in detail. It should be understood, however, that it is not intended to limit the present disclosure to the particular forms disclosed, but on the contrary, the present disclosure is to cover all modifications, equivalents, and alternatives falling within the spirit and scope of the present disclosure as defined by the appended claims.

## DETAILED DESCRIPTION

The present disclosure is described with reference to the attached figures, where like reference numerals are used throughout the figures to designate similar or equivalent elements. The figures are not drawn to scale, and are provided merely to illustrate the instant disclosure. Several aspects of the disclosure are described below with reference to example applications for illustration.

Many individuals suffer from sleep-related and/or respiratory disorders. Examples of sleep-related and/or respiratory disorders include Periodic Limb Movement Disorder (PLMD), Restless Leg Syndrome (RLS), Sleep-Disordered Breathing (SDB) such as Obstructive Sleep Apnea (OSA), Central Sleep Apnea (CSA), and other types of apneas such as mixed apneas and hypopneas, Respiratory Effort Related Arousal (RERA), Cheyne-Stokes Respiration (CSR), respiratory insufficiency, Obesity Hyperventilation Syndrome (OHS), Chronic Obstructive Pulmonary Disease (COPD), Neuromuscular Disease (NMD), rapid eye movement (REM) behavior disorder (also referred to as RBD), dream enactment behavior (DEB), hyper tension, diabetes, stroke, insomnia, and chest wall disorders.

Obstructive Sleep Apnea (OSA) is a form of Sleep Disordered Breathing (SDB), and is characterized by events including occlusion or obstruction of the upper air passage during sleep resulting from a combination of an abnormally small upper airway and the normal loss of muscle tone in the region of the tongue, soft palate and posterior oropharyngeal wall. More generally, an apnea generally refers to the cessation of breathing caused by blockage of the air (Obstructive Sleep Apnea) or the stopping of the breathing function (often referred to as Central Sleep Apnea). Typically, the individual will stop breathing for between about 15 seconds and about 30 seconds during an obstructive sleep apnea event.

Other types of apneas include hypopnea, hyperpnea, and hypercapnia. Hypopnea is generally characterized by slow or shallow breathing caused by a narrowed airway, as opposed to a blocked airway. Hyperpnea is generally characterized by an increase depth and/or rate of breathing. Hypercapnia is generally characterized by elevated or excessive carbon dioxide in the bloodstream, typically caused by inadequate respiration.

Cheyne-Stokes Respiration (CSR) is another form of sleep disordered breathing. CSR is a disorder of a patient's respiratory controller in which there are rhythmic alternating periods of waxing and waning ventilation known as CSR cycles. CSR is characterized by repetitive deoxygenation and re-oxygenation of the arterial blood.

Obesity Hyperventilation Syndrome (OHS) is defined as the combination of severe obesity and awake chronic hypercapnia, in the absence of other known causes for hypoventilation. Symptoms include dyspnea, morning headache and excessive daytime sleepiness.

Chronic Obstructive Pulmonary Disease (COPD) encompasses any of a group of lower airway diseases that have certain characteristics in common, such as increased resistance to air movement, extended expiratory phase of respiration, and loss of the normal elasticity of the lung.

Neuromuscular Disease (NMD) encompasses many diseases and ailments that impair the functioning of the muscles either directly via intrinsic muscle pathology, or indirectly via nerve pathology. Chest wall disorders are a group of thoracic deformities that result in inefficient coupling between the respiratory muscles and the thoracic cage.

A Respiratory Effort Related Arousal (RERA) event is typically characterized by an increased respiratory effort for ten seconds or longer leading to arousal from sleep and which does not fulfill the criteria for an apnea or hypopnea event. RERAs are defined as a sequence of breaths characterized by increasing respiratory effort leading to an arousal from sleep, but which does not meet criteria for an apnea or hypopnea. These events must fulfil both of the following criteria: (1) a pattern of progressively more negative esophageal pressure, terminated by a sudden change in pressure to a less negative level and an arousal, and (2) the event lasts ten seconds or longer. In some implementations, a Nasal Cannula/Pressure Transducer System is adequate and reliable in the detection of RERAs. A RERA detector may be based on a real flow signal derived from a respiratory therapy device. For example, a flow limitation measure may be determined based on a flow signal. A measure of arousal may then be derived as a function of the flow limitation measure and a measure of sudden increase in ventilation. One such method is described in WO 2008/138040 and U.S. Patent No. 9,358,353, assigned to ResMed Ltd.

These and other disorders are characterized by particular events (e.g., snoring, an apnea, a hypopnea, a restless leg, a sleeping disorder, choking, an increased heart rate, labored breathing, an asthma attack, an epileptic episode, a seizure, or any combination thereof) that occur when the individual is sleeping.

The Apnea-Hypopnea Index (AHI) is an index used to indicate the severity of sleep apnea during a sleep session.

The AHI is calculated by dividing the number of apnea and/or hypopnea events experienced by the user during the sleep session by the total number of hours of sleep in the sleep session. The event can be, for example, a pause in breathing that lasts for at least 10 seconds. An AHI that is less than 5 is considered normal. An AHI that is greater than or equal to 5, but less than 15 is considered indicative of mild sleep apnea. An AHI that is greater than or equal to 15, but less than 30 is considered indicative of moderate sleep apnea. An AHI that is greater than or equal to 30 is considered indicative of severe sleep apnea. In children, an AHI that is greater than 1 is considered abnormal. Sleep apnea can be considered "controlled" when the AHI is normal, or when the AHI is normal or mild. The AHI can also be used in combination with oxygen desaturation levels to indicate the severity of Obstructive Sleep Apnea.

Generally, activity levels while on respiratory therapy can be estimated directly from the flow signal alone. While the flow rate data gives some information regarding activity levels over a larger time frame, it does not identify movements with precise temporal resolution. Furthermore, features of the flow signal identifying regions of high activity could be confounded with other respiratory events, such as coughs, gasps, etc. Thus, aspects of the present disclosure relate to determining movements and/or activity level of the user using echo and/or audio data.

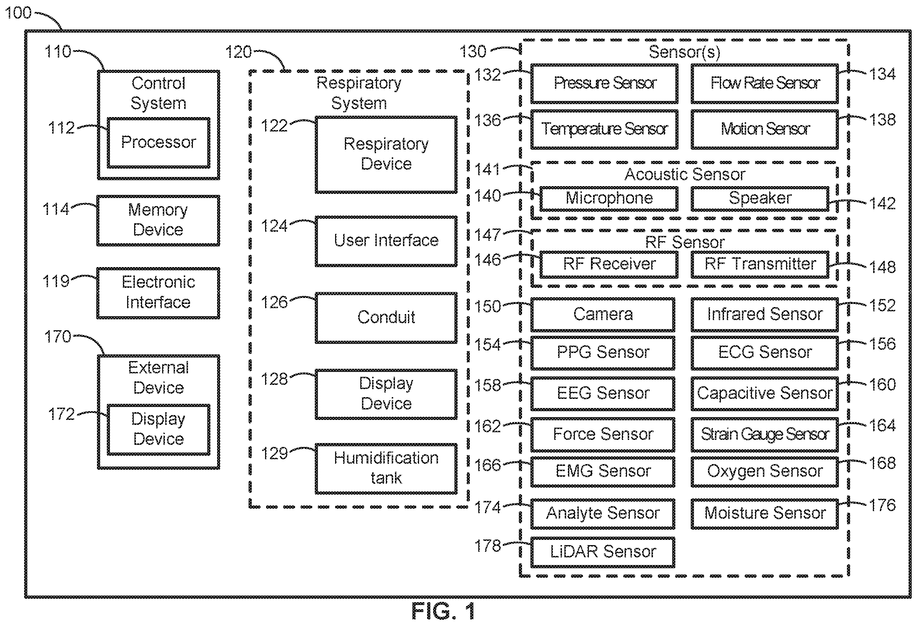

Referring to FIG. 1, a system 100, according to some implementations of the present disclosure, is illustrated. The system 100 is for providing a variety of different sensors related to a user's use of a respiratory therapy system, among other uses. The system 100 includes a control system 110, a memory device 114, an electronic interface 119, one or more sensors 130, and one or more user devices 170. In some implementation, the system 100 further includes a respiratory therapy system 120 that includes a respiratory therapy device 122. The system 100 can be used to determine movement of a user while using a respiratory therapy system and/or a body position of the user during a respiratory therapy session, as disclosed in further detail herein. The user's body position can include, for example, lying in a supine position, lying in a prone position, lying on his/her left side or right side, etc.

The control system 110 includes one or more processors 112 (hereinafter, processor 112). The control system 110 is generally used to control (e.g., actuate) the various components of the system 100 and/or analyze data obtained and/or generated by the components of the system 100. The processor 112 can be a general or special purpose processor or microprocessor. While one processor 112 is shown in FIG. 1, the control system 110 can include any suitable number of processors (e.g., one processor, two processors, five processors, ten processors, etc.) that can be in a single housing, or located remotely from each other. The control system 110 (or any other control system) or a portion of the control system 110 such as the processor 112 (or any other processor(s) or portion(s) of any other control system), can be used to carry out one or more steps of any of the methods described and/or claimed herein. The control system 110 can be coupled to and/or positioned within, for example, a housing of the user device 170, and/or within a housing of one or more of the sensors 130. The control system 110 can be centralized (within one such housing) or decentralized (within two or more of such housings, which are physically distinct). In such implementations including two or more housings containing the control system 110, such housings can be located proximately and/or remotely from each other.

The memory device 114 stores machine-readable instructions that are executable by the processor 112 of the control system 110. The memory device 114 can be any suitable computer readable storage device or media, such as, for example, a random or serial access memory device, a hard drive, a solid state drive, a flash memory device, etc. While one memory device 114 is shown in FIG. 1, the system 100 can include any suitable number of memory devices 114 (e.g., one memory device, two memory devices, five memory devices, ten memory devices, etc.). The memory device 114 can be coupled to and/or positioned within a housing of the respiratory therapy device 122, within a housing of the user device 170, within a housing of one or more of the sensors 130, or any combination thereof. Like the control system 110, the memory device 114 can be centralized (within one such housing) or decentralized (within two or more of such housings, which are physically distinct).

In some implementations, the memory device 114 stores a user profile associated with the user. The user profile can include, for example, demographic information associated with the user, biometric information associated with the user, medical information associated with the user, self-reported user feedback, sleep parameters associated with the user (e.g., sleep-related parameters recorded from one or more earlier sleep sessions), or any combination thereof. The demographic information can include, for example, information indicative of an age of the user, a gender of the user, a race of the user, a geographic location of the user, a relationship status, a family history of insomnia or sleep apnea, an employment status of the user, an educational status of the user, a socioeconomic status of the user, or any combination thereof. The medical information can include, for example, including indicative of one or more medical conditions associated with the user, medication usage by the user, or both. The medical information can also include a fall risk assessment associated with the user (e.g., a fall risk score using the Morse fall scale). The medical information can include, for example, information indicative of one or more medical conditions associated with the user, medication usage by the user, or both. The medical information data can further include a multiple sleep latency test (MSLT) result or score and/or a Pittsburgh Sleep Quality Index (PSQI) score or value. The medical information data can further include a self-reported subjective sleep score (e.g., poor, average, excellent), a self-reported subjective stress level of the user, a self-reported subjective fatigue level of the user, a self-reported subjective health status of the user, a recent life event experienced by the user, or any combination thereof. In some implementations, the memory device 114 stores media content that can be displayed on the display device 128.

The electronic interface 119 is configured to receive data (e.g., physiological data) from the one or more sensors 130 such that the data can be stored in the memory device 114 and/or analyzed by the processor 112 of the control system 110. The electronic interface 119 can communicate with the one or more sensors 130 using a wired connection or a wireless connection (e.g., using an RF communication protocol, a WiFi communication protocol, a Bluetooth communication protocol, an IR communication protocol, over a cellular network, over any other optical communication protocol, etc.). The electronic interface 119 can include an antenna, a receiver (e.g., an RF receiver), a transmitter (e.g., an RF transmitter), a transceiver, or any combination thereof. The electronic interface 119 can also include one more processors and/or one more memory devices that are the same as, or similar to, the processor 112 and the memory device 114 described herein. In some implementations, the electronic interface 119 is coupled to or integrated in the user device 170. In other implementations, the electronic interface 119 is coupled to or integrated (e.g., in a housing) with the control system 110 and/or the memory device 114.

The respiratory therapy system 120 includes a respiratory pressure therapy device (referred to as the respiratory therapy device 122), a user interface 124, a conduit 126 (also referred to as a tube or an air circuit), and optionally a display device 128, a humidification tank 129, or any combination thereof. In some implementations, the control system 110, the memory device 114, the display device 128, one or more of the sensors 130, and the humidification tank 129 are part of the respiratory therapy device 122. Respiratory pressure therapy refers to the application of a supply of air to an entrance to a user's airways at a controlled target pressure that is nominally positive with respect to atmosphere throughout the user's breathing cycle (e.g., in contrast to negative pressure therapies such as the tank ventilator or cuirass). The respiratory therapy system 120 is generally used to treat individuals suffering from one or more sleep-related respiratory disorders (e.g., obstructive sleep apnea, central sleep apnea, or mixed sleep apnea).

The respiratory therapy device 122 is generally used to generate pressurized air that is delivered to a user (e.g., using one or more motors that drive one or more compressors). In some implementations, the respiratory therapy device 122 generates continuous constant air pressure that is delivered to the user. In other implementations, the respiratory therapy device 122 generates two or more predetermined pressures (e.g., a first predetermined air pressure and a second predetermined air pressure). In still other implementations, the respiratory therapy device 122 is configured to generate a variety of different air pressures within a predetermined range. For example, the respiratory therapy device 122 can deliver at least about 6 $cmH_2O$, at least about 10 $cmH_2O$, at least about 20 $cmH_2O$, between about 6 $cmH_2O$ and about 10 $cmH_2O$, between about 7 $cmH_2O$ and about 12 $cmH_2O$, etc. The respiratory therapy device 122 can also deliver pressurized air at a predetermined flow rate between, for example, about -20 L/min and about 150 L/min, while maintaining a positive pressure (relative to the ambient pressure).

The user interface 124 engages a portion of the user's face and delivers pressurized air from the respiratory therapy device 122 to the user's airway to aid in preventing the airway from narrowing and/or collapsing during sleep. This may also increase the user's oxygen intake during sleep. Generally, the user interface 124 engages the user's face such that the pressurized air is delivered to the user's airway via the user's mouth, the user's nose, or both the user's mouth and nose. Together, the respiratory therapy device 122, the user interface 124, and the conduit 126s form an air pathway fluidly coupled with an airway of the user. The pressurized air also increases the user's oxygen intake during sleep. Depending upon the therapy to be applied, the user interface 124 may form a seal, for example, with a region or portion of the user's face, to facilitate the delivery of gas at a pressure at sufficient variance with ambient pressure to effect therapy, for example, at a positive pressure of about 10 $cmH_2O$ relative to ambient pressure. For other forms of therapy, such as the delivery of oxygen, the user interface may not include a seal sufficient to facilitate delivery to the airways of a supply of gas at a positive pressure of about 10 $cmH_2O$.

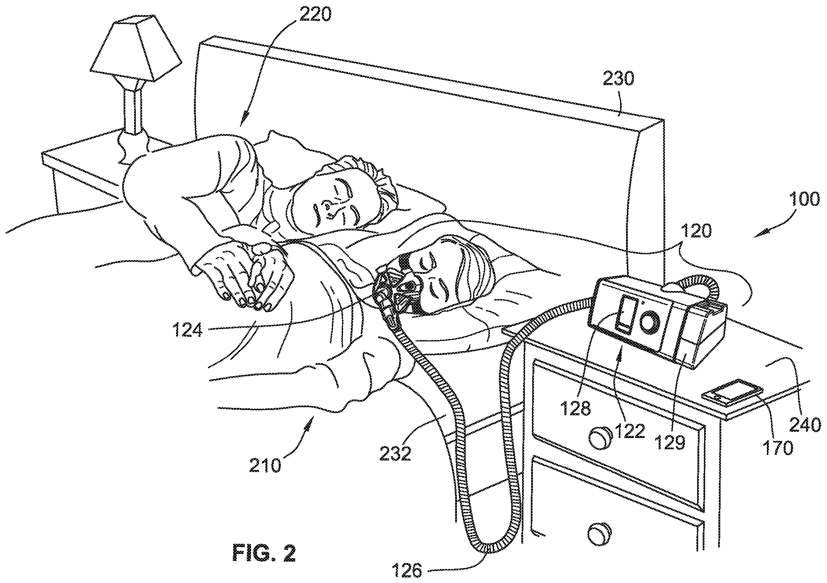

As shown in FIG. 2, in some implementations, the user interface 124 is or includes a facial mask (e.g., a full face mask) that covers at least a portion of the nose and mouth of the user 210. Alternatively, in some implementations, the user interface 124 can be a nasal mask that provides air to the nose of the user or a nasal pillow mask that delivers air directly to the nostrils of the user. The user interface 124 can include a plurality of straps (e.g., including hook and loop fasteners) for positioning and/or stabilizing the interface on a portion of the user (e.g., the face) and a conformal cushion (e.g., silicone, plastic, foam, etc.) that aids in providing an air-tight seal between the user interface 124 and the user. The user interface 124 can also include one or more vents for permitting the escape of carbon dioxide and other gases exhaled by the user 210. In other implementations, the user interface 124 includes a mouthpiece (e.g., a night guard mouthpiece molded to conform to the user's teeth, a mandibular repositioning device, etc.).

The conduit 126 (also referred to as an air circuit or tube) allows the flow of air between components of the respiratory therapy system 120, such as between the respiratory therapy device 122 and the user interface 124. In some implementations, there can be separate limbs of the conduit for inhalation and exhalation. In other implementations, a single limb conduit is used for both inhalation and exhalation.

One or more of the respiratory therapy device 122, the user interface 124, the conduit 126, the display device 128, and the humidification tank 129 can contain one or more sensors (e.g., a pressure sensor, a flow rate sensor, or more generally any of the other sensors 130 described herein). These one or more sensors can be use, for example, to measure the air pressure and/or flow rate of pressurized air supplied by the respiratory therapy device 122.

The display device 128 is generally used to display image(s) including still images, video images, or both and/or information regarding the respiratory therapy device 122. For example, the display device 128 can provide information regarding the status of the respiratory therapy device 122 (e.g., whether the respiratory therapy device 122 is on/off, the pressure of the air being delivered by the respiratory therapy device 122, the temperature of the air being delivered by the respiratory therapy device 122, etc.) and/or other information (e.g., a sleep score and/or a therapy score, also referred to as a myAir™ score, such as described in WO 2016/061629 and U.S. Patent Pub. No. 2017/0311879, the current date/time, personal information for the user 210, etc.). In some implementations, the display device 128 acts as a human-machine interface (HMI) that includes a graphic user interface (GUI) configured to display the image(s) as an input interface. The display device 128 can be an LED display, an OLED display, an LCD display, or the like. The input interface can be, for example, a touchscreen or touch-sensitive substrate, a mouse, a keyboard, or any sensor system configured to sense inputs made by a human user interacting with the respiratory therapy device 122.

The humidification tank 129 is coupled to or integrated in the respiratory therapy device 122 and includes a reservoir for storing water that can be used to humidify the pressurized air delivered from the respiratory therapy device 122. The respiratory therapy device 122 can include a heater to heat the water in the humidification tank 129 in order to humidify the pressurized air provided to the user. Additionally, in some implementations, the conduit 126 can also include a heating element (e.g., coupled to and/or imbedded in the conduit 126) that heats the pressurized air delivered to the user. The humidification tank 129 can be fluidly coupled to a water vapor inlet of the air pathway and deliver water vapor into the air pathway via the water vapor inlet, or can be formed in-line with the air pathway as part of the air pathway itself. In other implementations, the respiratory therapy device 122 or the conduit 126 can include a waterless humidifier. The waterless humidifier can incorporate sensors that interface with other sensor positioned elsewhere in system 100.

In some implementations, the system 100 can be used to deliver at least a portion of a substance from the receptacle 180 to the air pathway the user based at least in part on the physiological data, the sleep-related parameters, other data or information, or a combination thereof. Generally, modifying the delivery of the portion of the substance into the air pathway can include (i) initiating the delivery of the substance into the air pathway, (ii) ending the delivery of the portion of the substance into the air pathway, (iii) modifying an amount of the substance delivered into the air pathway, (iv) modifying a temporal characteristic of the delivery of the portion of the substance into the air pathway, (v) modifying a quantitative characteristic of the delivery of the portion of the substance into the air pathway, (vi) modifying any parameter associated with the delivery of the substance into the air pathway, or (vii) a combination of (i)-(vi).

Modifying the temporal characteristic of the delivery of the portion of the substance into the air pathway can include changing the rate at which the substance is delivered, starting and/or finishing at different times, continuing for different time periods, changing the time distribution or characteristics of the delivery, changing the amount distribution independently of the time distribution, etc. The independent time and amount variation ensures that, apart from varying the frequency of the release of the substance, one can vary the amount of substance released each time. In this manner, a number of different combination of release frequencies and release amounts (e.g., higher frequency but lower release amount, higher frequency and higher amount, lower frequency and higher amount, lower frequency and lower amount, etc.) can be achieved. Other modifications to the delivery of the portion of the substance into the air pathway can also be utilized.

The respiratory therapy system 120 can be used, for example, as a ventilator or a positive airway pressure (PAP) system, such as a continuous positive airway pressure (CPAP) system, an automatic positive airway pressure system (APAP), a bi-level or variable positive airway pressure system (BPAP or VPAP), or any combination thereof. The CPAP system delivers a predetermined air pressure (e.g., determined by a sleep physician) to the user. The APAP system automatically varies the air pressure delivered to the user based on, for example, respiration data associated with the user. The BPAP or VPAP system is configured to deliver a first predetermined pressure (e.g., an inspiratory positive airway pressure or IPAP) and a second predetermined pressure (e.g., an expiratory positive airway pressure or EPAP) that is lower than the first predetermined pressure.

Still referring to FIG. 1, the one or more sensors 130 of the system 100 include a pressure sensor 132, a flow rate sensor 134, a temperature sensor 136, a motion sensor 138, the microphone 140, the speaker 142, a radio-frequency (RF) receiver 146, a RF transmitter 148, a camera 150, an infrared sensor 152, a photoplethysmogram (PPG) sensor 154, an electrocardiogram (ECG) sensor 156, an electroencephalography (EEG) sensor 158, a capacitive sensor 160, a force sensor 162, a strain gauge sensor 164, an electromyography (EMG) sensor 166, an oxygen sensor 168, an analyte sensor 174, a moisture sensor 176, a LiDAR sensor 178, or a combination thereof. Generally, each of the one or more sensors 130 are configured to output sensor data that is received and stored in the memory device 114 or one or more other memory devices.

While the one or more sensors 130 are shown and described as including each of the pressure sensor 132, the flow rate sensor 134, the temperature sensor 136, the motion sensor 138, the microphone 140, the speaker 142, the RF receiver 146, the RF transmitter 148, the camera 150, the IR sensor 152, the PPG sensor 154, the ECG sensor 156, the EEG sensor 158, the capacitive sensor 160, the force sensor 162, the strain gauge sensor 164, the EMG sensor 166, the oxygen sensor 168, the analyte sensor 174, the moisture sensor 176, and the LidAR sensor 178, more generally, the one or more sensors 130 can include any combination and any number of each of the sensors described and/or shown herein.

As described herein, the system 100 generally can be used to generate physiological data associated with a user (e.g., a user of the respiratory therapy system 120 shown in FIG. 2) during a sleep session. The physiological data can be analyzed to generate one or more sleep-related parameters, which can include any parameter, measurement, etc. related to the user during the sleep session. The one or more sleep-related parameters that can be determined for the user 210 during the sleep session include, for example, an Apnea-Hypopnea Index (AHI) score, a sleep score, a flow signal, a respiration signal, a respiration rate, an inspiration amplitude, an expiration amplitude, an inspiration-expiration ratio, a number of events per hour, a pattern of events, a stage, pressure settings of the respiratory therapy device 122, a heart rate, a heart rate variability, movement of the user 210, temperature, EEG activity, EMG activity, arousal, snoring, choking, coughing, whistling, wheezing, or a combination thereof.

In some implementations, the physiological data generated by one or more of the sensors 130 can be used by the control system 110 to determine a sleep-wake signal associated with a user during the sleep session and one or more sleep-related parameters. The sleep-wake signal can be indicative of one or more sleep states, including sleep, wakefulness, relaxed wakefulness, micro-awakenings, or distinct sleep stages such as a rapid eye movement (REM) stage, a first non-REM stage (often referred to as "N1"), a second non-REM stage (often referred to as "N2"), a third non-REM stage (often referred to as "N3"), or any combination thereof. Methods for determining sleep states and/or sleep stages from physiological data generated by one or more sensors, such as the one or more sensors 130, are described in, for example, WO 2014/047310, U.S. Patent Pub. No. 2014/0088373, WO 2017/132726, WO 2019/122413, WO 2019/122414, and U.S. Patent Pub. No. 2020/0383580.

The sleep-wake signal can also be timestamped to determine a time that the user enters the bed, a time that the user exits the bed, a time that the user attempts to fall asleep, etc. The sleep-wake signal can be measured by the one or more sensors 130 during the sleep session at a predetermined sampling rate, such as, for example, one sample per second, one sample per 30 seconds, one sample per minute, etc. In some implementations, the sleep-wake signal can also be indicative of a respiration signal, a respiration rate, an inspiration amplitude, an expiration amplitude, an inspiration-expiration ratio, a number of events per hour, a pattern of events, pressure settings of the respiratory therapy device 122, or any combination thereof during the sleep session. The event(s) can include snoring, apneas, central apneas, obstructive apneas, mixed apneas, hypopneas, a mouth leak, a mask leak (e.g., from the user interface 124), a restless leg, a sleeping disorder, choking, an increased heart rate, a heart rate variation, labored breathing, an asthma attack, an epileptic episode, a seizure, a fever, a cough, a sneeze, a snore, a gasp, the presence of an illness such as the common cold or the flu, or any combination thereof. The one or more sleep-related parameters that can be determined for the user during the sleep session based on the sleep-wake signal include, for example, sleep quality metrics such as a total time in bed, a total sleep time, a sleep onset latency, a wake-after-sleep-onset parameter, a sleep efficiency, a fragmentation index, or any combination thereof. As described in further detail herein, the physiological data and/or the sleep-related parameters can be analyzed to determine one or more sleep-related scores.

Physiological data and/or audio data generated by the one or more sensors 130 can also be used to determine a respiration signal associated with a user during a sleep session. The respiration signal is generally indicative of respiration or breathing of the user during the sleep session. The respiration signal can be indicative of and/or analyzed to determine (e.g., using the control system 110) one or more sleep-related parameters, such as, for example, a respiration rate, a respiration rate variability, an inspiration amplitude, an expiration amplitude, an inspiration-expiration ratio, an occurrence of one or more events, a number of events per hour, a pattern of events, a sleep state, a sleet stage, an apnea-hypopnea index (AHI), pressure settings of the respiratory therapy device 122, or any combination thereof. The one or more events can include snoring, apneas, central apneas, obstructive apneas, mixed apneas, hypopneas, a mask leak (e.g., from the user interface 124), a cough, a restless leg, a sleeping disorder, choking, an increased heart rate, labored breathing, an asthma attack, an epileptic episode, a seizure, increased blood pressure, or any combination thereof. Many of the described sleep-related parameters are physiological parameters, although some of the sleep-related parameters can be considered to be non-physiological parameters. Other types of physiological and/or non-physiological parameters can also be determined, either from the data from the one or more sensors 130, or from other types of data.

Generally, the sleep session includes any point in time after the user 210 has laid or sat down in the bed 230 (or another area or object on which they intend to sleep), and has turned on the respiratory therapy device 122 and donned the user interface 124. The sleep session can thus include time periods (i) when the user 210 is using the CPAP system but before the user 210 attempts to fall asleep (for example when the user 210 lays in the bed 230 reading a book); (ii) when the user 210 begins trying to fall asleep but is still awake; (iii) when the user 210 is in a light sleep (also referred to as stage 1 and stage 2 of non-rapid eye movement (NREM) sleep); (iv) when the user 210 is in a deep sleep (also referred to as slow-wave sleep, SWS, or stage 3 of NREM sleep); (v) when the user 210 is in rapid eye movement (REM) sleep; (vi) when the user 210 is periodically awake between light sleep, deep sleep, or REM sleep; or (vii) when the user 210 wakes up and does not fall back asleep.

The sleep session is generally defined as ending once the user 210 removes the user interface 124, turns off the respiratory therapy device 122, and gets out of bed 230. In some implementations, the sleep session can include additional periods of time, or can be limited to only some of the above-disclosed time periods. For example, the sleep session can be defined to encompass a period of time beginning when the respiratory therapy device 122 begins supplying the pressurized air to the airway or the user 210, ending when the respiratory therapy device 122 stops supplying the pressurized air to the airway of the user 210, and including some or all of the time points in between, when the user 210 is asleep or awake.

The pressure sensor 132 outputs pressure data that can be stored in the memory device 114 and/or analyzed by the processor 112 of the control system 110. In some implementations, the pressure sensor 132 is an air pressure sensor (e.g., barometric pressure sensor) that generates sensor data indicative of the respiration (e.g., inhaling and/or exhaling) of the user of the respiratory therapy system 120 and/or ambient pressure. In such implementations, the pressure sensor 132 can be coupled to or integrated in the respiratory therapy device 122, the user interface 124, or the conduit 126. The pressure sensor 132 can be used to determine an air pressure in the respiratory therapy device 122, an air pressure in the conduit 126, an air pressure in the user interface 124, or any combination thereof. The pressure sensor 132 can be, for example, a capacitive sensor, an electromagnetic sensor, an inductive sensor, a resistive sensor, a piezoelectric sensor, a strain-gauge sensor, an optical sensor, a potentiometric sensor, or any combination thereof. In some examples, the pressure sensor 132 can be used to determine a blood pressure of a user.

The flow rate sensor 134 outputs flow rate data that can be stored in the memory device 114 and/or analyzed by the processor 112 of the control system 110. Examples of flow rate sensors (such as, for example, the flow rate sensor 214) are described in International Publication No. WO 2012/012835 and U.S. Patent No. 10,328,219. In some implementations, the flow rate sensor 134 is used to determine an air flow rate from the respiratory therapy device 122, an air flow rate through the conduit 126, an air flow rate through the user interface 124, or any combination thereof. In such implementations, the flow rate sensor 134 can be coupled to or integrated in the respiratory therapy device 122, the user interface 124, or the conduit 126. The flow rate sensor 134 can be a mass flow rate sensor such as, for example, a rotary flow meter (e.g., Hall effect flow meters), a turbine flow meter, an orifice flow meter, an ultrasonic flow meter, a hot wire sensor, a vortex sensor, a membrane sensor, or any combination thereof. In some implementations, the flow rate sensor 214 is configured to measure a vent flow (e.g., intentional "leak"), an unintentional leak (e.g., mouth leak and/or mask leak), a patient flow (e.g., air into and/or out of lungs), or any combination thereof. In some implementations, the flow rate data can be analyzed to determine cardiogenic oscillations of the user.

The temperature sensor 136 outputs temperature data that can be stored in the memory device 114 and/or analyzed by the processor 112 of the control system 110. In some implementations, the temperature sensor 136 generates temperature data indicative of a core body temperature of the user 210 (FIG. 2), a skin temperature of the user 210, a temperature of the air flowing from the respiratory therapy device 122 and/or through the conduit 126, a temperature of the air in the user interface 124, an ambient temperature, or any combination thereof. The temperature sensor 136 can be, for example, a thermocouple sensor, a thermistor sensor, a silicon band gap temperature sensor or semiconductor-based sensor, a resistance temperature detector, or any combination thereof.

The motion sensor 138 outputs motion data that can be stored in the memory device 114 and/or analyzed by the processor 112 of the control system 110. The motion sensor 138 can be used to detect movement of the user 210 during the sleep session, and/or detect movement of any of the components of the respiratory therapy system 120, such as the respiratory therapy device 122, the user interface 124, or the conduit 126. The motion sensor 138 can include one or more inertial sensors, such as accelerometers, gyroscopes, and magnetometers. In some implementations, the motion sensor 138 alternatively or additionally generates one or more signals representing bodily movement of the user, from which may be obtained a signal representing a sleep state of the user; for example, via a respiratory movement of the user. In some implementations, the motion data from the motion sensor 138 can be used in conjunction with additional data from another sensor 130 to determine the sleep state of the user.

The microphone 140 outputs sound data that can be stored in the memory device 114 and/or analyzed by the processor 112 of the control system 110. The audio data generated by the microphone 140 is reproducible as one or more sound(s) during a sleep session (e.g., sounds from the user 210). The audio data from the microphone 140 can also be used to identify (e.g., using the control system 110) an event experienced by the user during the sleep session, as described in further detail herein. The microphone 140 can be coupled to or integrated with (e.g., on an internal or external surface of) the respiratory therapy device 122, the user interface 124, the conduit 126, or the user device 170. In some implementations, the system 100 includes a plurality of microphones (e.g., two or more microphones and/or an array of microphones with beamforming) such that sound data generated by each of the plurality of microphones can be used to discriminate the sound data generated by another of the plurality of microphones.

The speaker 142 can output sound waves that are audible to a user of the system 100 (e.g., the user 210 of FIG. 2) or inaudible to the user of the system (e.g., ultrasonic sound waves). The speaker 142 can be used, for example, as an alarm clock or to play an alert or message to the user 210 or caregiver (e.g., in response to a movement event and/or change in body position). In some implementations, the speaker 142 can be used to communicate the audio data generated by the microphone 140 to the user. The speaker 142 can be coupled to or integrated in the respiratory therapy device 122, the user interface 124, the conduit 126, or the external device 170.

The microphone 140 and the speaker 142 can be used as separate devices. In some implementations, the microphone 140 and the speaker 142 can be combined into an acoustic sensor 141 (e.g., a SONAR sensor), as described in, for example, WO 2018/050913 and WO 2020/104465. In such implementations, the speaker 142 generates or emits sound waves at a predetermined interval and the microphone 140 detects the reflections of the emitted sound waves from the speaker 142. In one or more implementations, the sound waves generated or emitted by the speaker 142 can have a frequency that is not audible to the human ear (e.g., below 20 Hz or above around 18 kHz) so as not to disturb the sleep of the user 210 or the bed partner 220 (FIG. 2). Based at least in part on the data from the microphone 140 and/or the speaker 142, the control system 110 can determine a location of the user 210 (FIG. 2) and/or one or more of the sleep-related parameters described in herein such as, for example, a movement, a body position, a respiration signal, a respiration rate, an inspiration amplitude, an expiration amplitude, an inspiration-expiration ratio, a number of events per hour, a pattern of events, a sleep state, a sleep stage, pressure settings of the respiratory therapy device 122, or any combination thereof. In such a context, a sonar sensor may be understood to concern an active acoustic sensing, such as by generating and/or transmitting ultrasound and/or low frequency ultrasound sensing signals (e.g., in a frequency range of about 17-23 kHz, 18-22 kHz, or 17-18 kHz, for example), through the air.

In some implementations, the sensors 130 include (i) a first microphone that is the same as, or similar to, the microphone 140, and is integrated in the acoustic sensor 141 and (ii) a second microphone that is the same as, or similar to, the microphone 140, but is separate and distinct from the first microphone that is integrated in the acoustic sensor 141.

The RF transmitter 148 generates and/or emits radio waves having a predetermined frequency and/or a predetermined amplitude (e.g., within a high frequency band, within a low frequency band, long wave signals, short wave signals, etc.). The RF receiver 146 detects the reflections of the radio waves emitted from the RF transmitter 148, and this data can be analyzed by the control system 110 to determine a location and/or movement, such as respiratory and/or bodily movement, of the user 210 (FIG. 2) and/or one or more of the sleep-related parameters described herein. An RF receiver (either the RF receiver 146 and the RF transmitter 148 or another RF pair) can also be used for wireless communication between the control system 110, the respiratory therapy device 122, the one or more sensors 130, the user device 170, or a combination thereof. While the RF receiver 146 and RF transmitter 148 are shown as being separate and distinct elements in FIG. 1, in some implementations, the RF receiver 146 and the RF transmitter 148 are combined as a part of an RF sensor 147 (e.g., a RADAR sensor). In some such implementations, the RF sensor 147 includes a control circuit. The specific format of the RF communication can be Wi-Fi, Bluetooth, or the like.

In some implementations, the RF sensor 147 is a part of a mesh system. One example of a mesh system is a WiFi mesh system, which can include mesh nodes, mesh router(s), and mesh gateway(s), each of which can be mobile/movable or fixed. In such implementations, the WiFi mesh system includes a WiFi router and/or a WiFi controller and one or more satellites (e.g., access points), each of which include an RF sensor that the is the same as, or similar to, the RF sensor 147. The WiFi router and satellites continuously communicate with one another using WiFi signals. The WiFi mesh system can be used to generate motion data based on changes in the WiFi signals (e.g., differences in received signal strength) between the router and the satellite(s) due to an object or person moving partially obstructing the signals. The motion data can be indicative of motion, breathing, heart rate, gait, falls, behavior, etc., or any combination thereof.

The camera 150 outputs image data reproducible as one or more images (e.g., still images, video images, thermal images, or a combination thereof) that can be stored in the memory device 114. The image data from the camera 150 can be used by the control system 110 to determine one or more of the sleep-related parameters described herein. The image data from the camera 150 can be used by the control system 110 to determine one or more of the sleep-related parameters described herein, such as, for example, one or more events (e.g., periodic limb movement or restless leg syndrome), a respiration signal, a respiration rate, an inspiration amplitude, an expiration amplitude, an inspiration-expiration ratio, a number of events per hour, a pattern of events, a sleep state, a sleep stage, or a combination thereof. Further, the image data from the camera 150 can be used to identify a location of the user, to determine chest movement of the user 210, to determine air flow of the mouth and/or nose of the user 210, to determine a time when the user 210 enters the bed 230, and to determine a time when the user 210 exits the bed 230. The camera 150 can also be used to track eye movements, pupil dilation (if one or both of the user 210's eyes are open), blink rate, or any changes during REM sleep. In some implementations, the camera 150 includes a wide angle lens or a fish eye lens.

The infrared (IR) sensor 152 outputs infrared image data reproducible as one or more infrared images (e.g., still images, video images, or both) that can be stored in the memory device 114. The infrared data from the IR sensor 152 can be used to determine one or more sleep-related parameters during the sleep session, including a temperature of the user 210 and/or movement of the user 210. The IR sensor 152 can also be used in conjunction with the camera 150 when measuring the presence, location, and/or movement of the user 210. The IR sensor 152 can detect infrared light having a wavelength between about 700 nm and about 1 mm, for example, while the camera 150 can detect visible light having a wavelength between about 380 nm and about 740 nm.

The PPG sensor 154 outputs physiological data associated with the user 210 (FIG. 2) that can be used to determine one or more sleep-related parameters, such as, for example, a heart rate, a heart rate pattern, a heart rate variability, a cardiac cycle, respiration rate, an inspiration amplitude, an expiration amplitude, an inspiration-expiration ratio, estimated blood pressure parameter(s), or any combination thereof. The PPG sensor 154 can be worn by the user 210, embedded in clothing and/or fabric that is worn by the user 210, embedded in and/or coupled to the user interface 124 and/or its associated headgear (e.g., straps, etc.), etc.

The ECG sensor 156 outputs physiological data associated with electrical activity of the heart of the user 210 (FIG. 2). In some implementations, the ECG sensor 156 includes one or more electrodes that are positioned on or around a portion of the user 210 during the sleep session. The physiological data from the ECG sensor 156 can be used, for example, to determine one or more of the sleep-related parameters described herein.

The EEG sensor 158 outputs physiological data associated with electrical activity of the brain of the user 210. In some implementations, the EEG sensor 158 includes one or more electrodes that are positioned on or around the scalp of the user 210 during the sleep session. The physiological data from the EEG sensor 158 can be used, for example, to determine a sleep state of the user 210 at any given time during the sleep session. In some implementations, the EEG sensor 158 can be integrated in the user interface 124 and/or the associated headgear (e.g., straps, etc.).

The capacitive sensor 160, the force sensor 162, and the strain gauge sensor 164 output data that can be stored in the memory device 114 and used by the control system 110 to determine one or more of the sleep-related parameters described herein. The EMG sensor 166 outputs physiological data associated with electrical activity produced by one or more muscles. The oxygen sensor 168 outputs oxygen data indicative of an oxygen concentration of gas (e.g., in the conduit 126 or at the user interface 124). The oxygen sensor 168 can be, for example, an ultrasonic oxygen sensor, an electrical oxygen sensor, a chemical oxygen sensor, an optical oxygen sensor, a pulse oximeter (e.g., $SpO_2$ sensor), or any combination thereof. In some implementations, the one or more sensors 130 also include a galvanic skin response (GSR) sensor, a blood flow sensor, a respiration sensor, a pulse sensor, a sphygmomanometer sensor, an oximetry sensor, or any combination thereof.

The analyte sensor 174 can be used to detect the presence of an analyte in the exhaled breath of the user 210. The data output by the analyte sensor 174 can be stored in the memory device 114 and used by the control system 110 to determine the identity and concentration of any analytes in the user 210's breath. In some implementations, the analyte sensor 174 is positioned near a mouth of the user 210 to detect analytes in breath exhaled from the user 210's mouth. For example, when the user interface 124 is a facial mask that covers the nose and mouth of the user 210, the analyte sensor 174 can be positioned within the facial mask to monitor the user 210's mouth breathing. In other implementations, such as when the user interface 124 is a nasal mask or a nasal pillow mask, the analyte sensor 174 can be positioned near the nose of the user 210 to detect analytes in breath exhaled through the user 210's nose. In still other implementations, the analyte sensor 174 can be positioned near the user 210's mouth when the user interface 124 is a nasal mask or a nasal pillow mask. In some implementations, the analyte sensor 174 can be used to detect whether any air is inadvertently leaking from the user 210's mouth. In some implementations, the analyte sensor 174 is a volatile organic compound (VOC) sensor that can be used to detect carbon-based chemicals or compounds. In some implementations, the analyte sensor 174 can also be used to detect whether the user 210 is breathing through their nose or mouth. For example, if the data output by an analyte sensor 174 positioned near the mouth of the user 210 or within the facial mask (in implementations where the user interface 124 is a facial mask) detects the presence of an analyte, the control system 110 can use this data as an indication that the user 210 is breathing through their mouth.

The moisture sensor 176 outputs data that can be stored in the memory device 114 and used by the control system 110. The moisture sensor 176 can be used to detect moisture in various areas surrounding the user (e.g., inside the conduit 126 or the user interface 124, near the user 210's face, near the connection between the conduit 126 and the user interface 124, near the connection between the conduit 126 and the respiratory therapy device 122, etc.). Thus, in some implementations, the moisture sensor 176 can be positioned in the user interface 124 or in the conduit 126 to monitor the humidity of the pressurized air from the respiratory therapy device 122. In other implementations, the moisture sensor 176 is placed near any area where moisture levels need to be monitored. The moisture sensor 176 can also be used to monitor the humidity of the ambient environment surrounding the user 210, for example the air inside the user 210's bedroom. The moisture sensor 176 can also be used to track the user 210's biometric response to environmental changes.

One or more Light Detection and Ranging (LiDAR) sensors 178 can be used for depth sensing. This type of optical sensor (e.g., laser sensor) can be used to detect objects and build three dimensional (3D) maps of the surroundings, such as of a living space. LiDAR can generally utilize a pulsed laser to make time of flight measurements. LiDAR is also referred to as 3D laser scanning. In an example of use of such a sensor, a fixed or mobile device (such as a smartphone) having a LiDAR sensor 178 can measure and map an area extending 5 meters or more away from the sensor. The LiDAR data can be fused with point cloud data estimated by an electromagnetic RADAR sensor, for example. The LiDAR sensor(s) 178 may also use artificial intelligence (AI) to automatically geofence RADAR systems by detecting and classifying features in a space that might cause issues for RADAR systems, such a glass windows (which can be highly reflective to RADAR). LiDAR can also be used to provide an estimate of the height of a person, as well as changes in height when the person sits down, or falls down, for example. LiDAR may be used to form a 3D mesh representation of an environment. In a further use, for solid surfaces through which radio waves pass (e.g., radio-translucent materials), the LiDAR may reflect off such surfaces, thus allowing a classification of different type of obstacles.

In some implementations, the one or more sensors 130 also include a galvanic skin response (GSR) sensor, a blood flow sensor, a respiration sensor, a pulse sensor, a sphygmomanometer sensor, an oximetry sensor, a sonar sensor, a RADAR sensor, a blood glucose sensor, a color sensor, a pH sensor, an air quality sensor, a tilt sensor, a rain sensor, a soil moisture sensor, a water flow sensor, an alcohol sensor, or any combination thereof.

While shown separately in FIG. 1, any combination of the one or more sensors 130 can be integrated in and/or coupled to any one or more of the components of the system 100, including the respiratory therapy device 122, the user interface 124, the conduit 126, the humidification tank 129, the control system 110, the user device 170, or any combination thereof. For example, the acoustic sensor 141 and/or the RF sensor 147 can be integrated in and/or coupled to the user device 170. In such implementations, the user device 170 can be considered a secondary device that generates additional or secondary data for use by the system 100 (e.g., the control system 110) according to some aspects of the present disclosure. In some implementations, at least one of the one or more sensors 130 is not physically and/or communicatively coupled to the respiratory therapy device 122, the control system 110, or the user device 170, and is positioned generally adjacent to the user 210 during the sleep session (e.g., positioned on or in contact with a portion of the user 210, worn by the user 210, coupled to or positioned on the nightstand, coupled to the mattress, coupled to the ceiling, etc.).

One or more of the respiratory therapy device 122, the user interface 124, the conduit 126, the display device 128, and the humidification tank 129 can contain one or more sensors (e.g., a pressure sensor, a flow rate sensor, or more generally any of the other sensors 130 described herein). These one or more sensors can be used, for example, to measure the air pressure and/or flow rate of pressurized air supplied by the respiratory therapy device 122.

The data from the one or more sensors 130 can be analyzed to determine one or more sleep-related parameters, which can include a respiration signal, a respiration rate, a respiration pattern, an inspiration amplitude, an expiration amplitude, an inspiration-expiration ratio, an occurrence of one or more events, a number of events per hour, a pattern of events, a sleep state, an apnea-hypopnea index (AHI), or any combination thereof. The one or more events can include snoring, apneas, central apneas, obstructive apneas, mixed apneas, hypopneas, an intentional mask leak, an unintentional mask leak, a mouth leak, a cough, a restless leg, a sleeping disorder, choking, an increased heart rate, labored breathing, an asthma attack, an epileptic episode, a seizure, increased blood pressure, or any combination thereof. Many of these sleep-related parameters are physiological parameters, although some of the sleep-related parameters can be considered to be non-physiological parameters. Non-physiological parameters can also include operational parameters of the respiratory therapy system, including flow rate, pressure, humidity of the pressurized air, speed of motor, etc. Other types of physiological and non-physiological parameters can also be determined, either from the data from the one or more sensors 130, or from other types of data.

The user device 170 (FIG. 1) includes a display device 172. The user device 170 can be, for example, a mobile device such as a smart phone, a tablet, a laptop, or the like. Alternatively, the user device 170 can be an external sensing system, a television (e.g., a smart television) or another smart home device (e.g., a smart speaker(s) such as Google Home, Amazon Echo, Alexa etc.). In some implementations, the user device is a wearable device (e.g., a smart watch). The display device 172 is generally used to display image(s) including still images, video images, or both. In some implementations, the display device 172 acts as a human-machine interface (HMI) that includes a graphic user interface (GUI) configured to display the image(s) and an input interface. The display device 172 can be an LED display, an OLED display, an LCD display, or the like. The input interface can be, for example, a touchscreen or touch-sensitive substrate, a mouse, a keyboard, or any sensor system configured to sense inputs made by a human user interacting with the user device 170. In some implementations, one or more user devices can be used by and/or included in the system 100.

While the control system 110 and the memory device 114 are described and shown in FIG. 1 as being a separate and distinct component of the system 100, in some implementations, the control system 110 and/or the memory device 114 are integrated in the user device 170 and/or the respiratory therapy device 122. Alternatively, in some implementations, the control system 110 or a portion thereof (e.g., the processor 112) can be located in a cloud (e.g., integrated in a server, integrated in an Internet of Things (IoT) device, connected to the cloud, be subject to edge cloud processing, etc.), located in one or more servers (e.g., remote servers, local servers, etc., or any combination thereof.

While the system 100 is shown as including all of the components described above, more or fewer components can be included in a system for analyzing data associated with a user's use of the respiratory therapy system 120, according to implementations of the present disclosure. For example, a first alternative system includes the control system 110, the memory device 114, and at least one of the one or more sensors 130 and does not include the respiratory therapy system 120. As another example, a second alternative system includes the control system 110, the memory device 114, at least one of the one or more sensors 130, and the user device 170. As yet another example, a third alternative system includes the control system 110, the memory device 114, the respiratory therapy system 120, at least one of the one or more sensors 130, and the user device 170. Thus, various systems for implementing the present disclosure can be formed using any portion or portions of the components shown and described herein and/or in combination with one or more other components.

Referring generally to FIG. 2, a portion of the system 100 (FIG. 1), according to some implementations, is illustrated. A user 210 of the respiratory therapy system 120 and a bed partner 220 are located in a bed 230 and are laying on a mattress 232. The user interface 124 can be worn by the user 210 during a sleep session. The user interface 124 is fluidly coupled and/or connected to the respiratory therapy device 122 via the conduit 126. In turn, the respiratory therapy device 122 delivers pressurized air to the user 210 via the conduit 126 and the user interface 124 to increase the air pressure in the throat of the user 210 to aid in preventing the airway from closing and/or narrowing during sleep. The respiratory therapy device 122 can be positioned on a nightstand 240 that is directly adjacent to the bed 230 as shown in FIG. 2, or more generally, on any surface or structure that is generally adjacent to the bed 230 and/or the user 210.

In some implementations, the control system 110, the memory 114, any of the one or more sensors 130, or a combination thereof can be located on and/or in any surface and/or structure that is generally adjacent to the bed 230 and/or the user 210. For example, in some implementations, at least one of the one or more sensors 130 can be located at a first position 255A on and/or in one or more components of the respiratory therapy system 120 adjacent to the bed 230 and/or the user 210. The one or more sensors 130 can be coupled to the respiratory therapy system 120, the user interface 124, the conduit 126, the display device 128, the humidification tank 129, or a combination thereof.

Alternatively, or additionally, at least one of the one or more sensors 130 can be located at a second position 255B on and/or in the bed 230 (e.g., the one or more sensors 130 are coupled to and/or integrated in the bed 230). Further, alternatively or additionally, at least one of the one or more sensors 130 can be located at a third position 255C on and/or in the mattress 232 that is adjacent to the bed 230 and/or the user 210 (e.g., the one or more sensors 130 are coupled to and/or integrated in the mattress 232). Alternatively, or additionally, at least one of the one or more sensors 130 can be located at a fourth position 255D on and/or in a pillow that is generally adjacent to the bed 230 and/or the user 210.

Alternatively, or additionally, at least one of the one or more sensors 130 can be located at a fifth position 255E on and/or in the nightstand 240 that is generally adjacent to the bed 230 and/or the user 210. Alternatively, or additionally, at least one of the one or more sensors 130 can be located at a sixth position 255F such that the at least one of the one or more sensors 130 are coupled to and/or positioned on the user 215 (e.g., the one or more sensors 130 are embedded in or coupled to fabric, clothing 212, and/or a smart device 270 worn by the user 210). More generally, at least one of the one or more sensors 130 can be positioned at any suitable location relative to the user 210 such that the one or more sensors 130 can generate sensor data associated with the user 210.

In some implementations, a primary sensor, such as the microphone 140, is configured to generate acoustic data associated with the user 210 during a sleep session. For example, one or more microphones (the same as, or similar to, the microphone 140 of FIG. 1) can be integrated in and/or coupled to (i) a circuit board of the respiratory therapy device 122, (ii) the conduit 126, (iii) a connector between components of the respiratory therapy system 120, (iv) the user interface 124, (v) a headgear (e.g., straps) associated with the user interface, or (vi) a combination thereof.

In some implementations, one or more secondary sensors may be used in addition to the primary sensor to generate additional data. In some such implementations, the one or more secondary sensors include: a microphone (e.g., the microphone 140 of the system 100), a flow rate sensor (e.g., the flow rate sensor 134 of the system 100), a temperature sensor (e.g., the temperature sensor 136 of the system 100), a camera (e.g., the camera 150 of the system 100), a vane sensor (VAF), a hot wire sensor (MAF), a cold wire, a laminar flow sensor, an ultrasonic sensor, an inertial sensor, or a combination thereof.

Additionally, or alternatively, one or more microphones (the same as, or similar to, the microphone 140 of FIG. 1) can be integrated in and/or coupled to a co-located smart device, such as the user device 170, a TV, a watch (e.g., a mechanical watch or the smart device 270), a pendant, the mattress 232, the bed 230, beddings positioned on the bed 230, the pillow, a speaker (e.g., the speaker 142 of FIG. 1), a radio, a tablet, a waterless humidifier, or a combination thereof.

Additionally, or alternatively, in some implementations, one or more microphones (the same as, or similar to, the microphone 140 of FIG. 1) can be remote from the system 100 (FIG. 1) and/or the user 210 (FIG. 2), so long as there is an air passage allowing acoustic signals to travel to the one or more microphones. For example, the one or more microphones can be in a different room from the room containing the system 100.

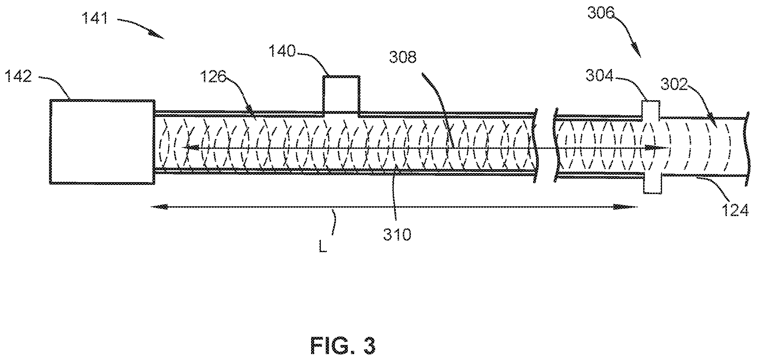

FIG. 3 illustrates the generation of acoustic data, in the form of echo data, in response to an acoustic reflection indicative of one or more features of a user interface (e.g., the user interface 124), according to aspects of the present disclosure. The generation of the echo data includes the acoustic sensor 141, which includes the microphone 140 (or any type of audio transducer) and a speaker 142. However, as discussed above, the speaker 142 can instead be replaced by another device that can generate an acoustic signal, such as the motor of the respiratory therapy device 122. The microphone 140 and speaker 142 are shown in specific locations relative to the conduit 126, which is connected to the respiratory therapy device 122 (not shown). However, the locations of the microphone 140 and the speaker 142 can vary from what is shown, as discussed above. Examples of generation and analysis of echo data are described in International Publication No. WO 2010/091462.

The speaker 142 emits an acoustic signal 302 within the conduit 126. The acoustic signal 302 is in the form of a sound. The sound can be one or more of a standard sound (e.g., an original, unmodified sound from an off-the-shelf sound application), a custom sound, an inaudible frequency, a white noise sound, a broad band impulse, a continuous sinusoidal waveform, a square waveform, a sawtooth waveform, and a frequency modulated sinusoid (e.g., chirp). According to some other implementations, the acoustic signal 302 can be in the form of one or more of an audible sound or an ultrasonic sound. According to some other implementations, the acoustic signal 302 is in the form of an inaudible sound, where the sound is inaudible based on one or both of the frequency of the sound (e.g., the frequency being outside of the frequency range for human hearing) or the amplitude of the sound (e.g., the amplitude being low enough that the sound is not loud enough for human perception).

In one or more implementations, the acoustic signal 302 is emitted at specific times, such as when the user first puts on the user interface, after the user takes off the user interface, after detecting that an apnea event or a hypopnea event is occurring (e.g., after detecting using a respiratory therapy device that an apnea event or a hypopnea event is occurring). For example, the specific monitoring times are selected to be at intervals of 0.1 seconds for a duration of at least 4 seconds. For continuous monitoring of a user, in determining movement and/or body position of a user, acoustic signal 302 may be emitted continuously or at intervals throughout all, or during one or more portions, of a therapy session.

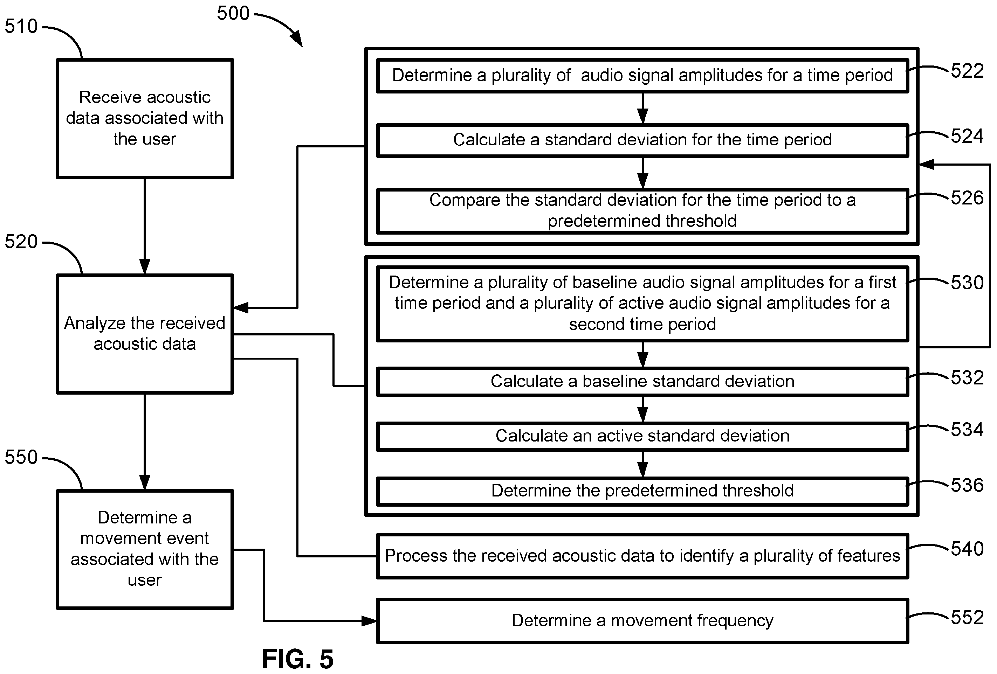

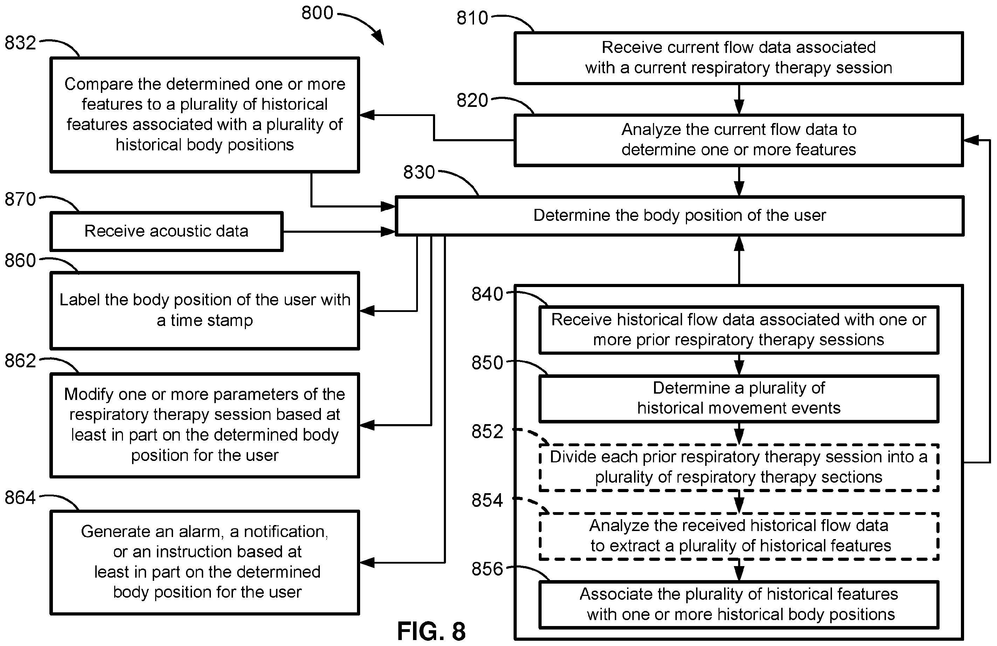

The acoustic signal 302 travels down the length L of the conduit 126 until the acoustic signal 302 contacts a feature 304 of the user interface 124 at or near a connection 306 of the user interface 124 with the conduit 126. The feature 304 includes a widening of the pathway 308 formed through the conduit 126 and/or the user interface 124. The widening at the feature 304 causes a change in the acoustic impedance of the acoustic signal 302 and an acoustic reflection 310. The acoustic reflection 310 travels back down the length L of the conduit 126 until it reaches the microphone 140. The microphone 140 detects the acoustic reflection 310 and generates echo data in response to the acoustic reflection 310. The generated data is subsequently analyzed for determining movement of the user and/or a body position of the user, as further discussed below with respect to, for example, FIG. 5 and/or FIG. 8.

The acoustic signal 302 can continue beyond the feature 304 and into the user interface 124. Although not illustrated, the user interface 124 and/or conduit 126 can include one or more additional features that change the acoustic impedance of the acoustic signal 302, which further generates the acoustic reflection 310. Thus, although only the feature 304 is illustrated and described as causing the acoustic reflection 310, there can be multiple features of the user interface 124 and/or conduit 126 that can all contribute to the acoustic reflection 310.

As such, in some implementations, the system 100 is configured to generate acoustic data, in the form of echo data, which is in turn analyzed by the control system 110. In some implementations, the echo data includes reflected sound waves received by a microphone (e.g., the microphone 140 of the system 100) that are transmitted from a speaker (e.g., the speaker 142 of the system 100, or an external speaker). The reflected sound waves are indicative of shapes and dimensions of the components in the sound waves' path(s). Additionally, or alternatively, the acoustic data includes audio data, such as sound(s) from the user that is indicative of one or more sleep-related parameters (e.g., breathing through the nose, breathing through the mouth, snoring, sniffling).

For example, the acoustic data (e.g., in the form of echo data) can include data generated by the microphone 140. The speaker 142 (or another device that can generate an acoustic signal, such as the motor of the respiratory therapy device 122) generates a sound. The sound can travel through the humidification tank 129, along a first connection, along the conduit 126, via a second connection, via a waterless humidifier (if fitted), to one or more cavities (e.g., nostrils and/or mouth), to the user's respiratory therapy system (including nose and/or mouth, airway(s), lungs, etc.). For each change or feature in the path (e.g., a cavity, a junction, a change in shape (e.g., due to movement of the conduit and/or other component of the respiratory therapy system), a change in location of the reflection (e.g., due to changes in effective length of the conduit during movement when the conduit is curled and/or stretched), and/or in length of the conduit which may be caused by movement of the conduit and/or another component of the respiratory therapy system), a reflection at that point is seen, the location of which may be determined based on the known speed of sound.

In some implementations, a cepstrum analysis is implemented to analyze the acoustic data. Cepstrum is a "quefrency" domain, which is also known as the spectrum of the log of a time domain waveform. For example, a cepstrum may be considered the inverse Fourier Transform of the log spectrum of the forward Fourier Transform of the decibel spectrum, etc. The operation essentially can convert a convolution of an impulse response function (IRF) and a sound source into an addition operation so that the sound source may then be more easily accounted for or removed so as to isolate data of the IRF for analysis. Techniques of cepstrum analysis are described in detail in a scientific paper entitled "The Cepstrum: A Guide to Processing" (Childers et al, Proceedings of the IEEE, Vol. 65, No. 10, Oct 1977) and Randall RB, Frequency Analysis, Copenhagen: Bruel & Kjaer, p. 344 (1977, revised ed. 1987).

Such a method may be understood in terms of the property of convolution. The convolution of f and g can be written as f * g. This operation can be the integral of the product of the two functions (f and g) after one is reversed and shifted. As such, it is a type of integral transform as Equation 1, as follows:

$$(f * g)(t) \overset{\text{def}}{=} \int_{-\infty}^{\infty} f(\tau) \cdot g(t - \tau) d\tau \qquad \text{Eq. 1}$$

While the symbol t is used above, it need not represent the time domain. But, in that context, the convolution formula can be described as a weighted average of the function f($\tau$) at the moment t where the weighting is given by g(-$\tau$)

simply shifted by amount t. As t changes, the weighting function emphasizes different parts of the input function.

More generally, if f and g are complex-valued functions on $R^d$, then their convolution may be defined as the integral of Equation 2:

$$(f * g)(x) = \int_{R^d} f(y)g(x-y)dy = \int_{R^d} f(x-y)g(y)dy \qquad \text{Eq. 2}$$

A mathematical model that can relate an acoustic system output to the input for a time-invariant linear system, such as one involving conduits of a respiratory treatment apparatus, (which may include some human or other unknown part of the system) can be based on this convolution. The output measured at a microphone of the system may be considered as the input noise "convolved" with the system Impulse Response Function (IRF) as a function of time (t), as shown in Equation 3:

$$y(t) = s_1(t) * h_1(t) \qquad \text{Eq. 3}$$

where * denotes the convolution function; y(t) is the signal measured at the sound sensor; $S_1(t)$ is the sound or noise source such, as a noise or sound created in or by a flow generator of a respiratory treatment apparatus; and $h_1(t)$ is the system IRF from the noise or sound source to the sound sensor. The Impulse Response Function (IRF) is the system response to a unit impulse input.

Conversion of Equation 3 into the frequency domain by means of the Fourier Transform of the measured sound data (e.g., a discrete Fourier Transform ("DFT") or a fast Fourier transform ("FFT") and considering the Convolution Theorem, Equation 4 is produced:

$$y(t) = s_1(t) * h_1(t) \xrightarrow{Fourier\ Transform} Y(f) = S_1(f)H_1(f) \qquad \text{Eq. 4}$$

where Y(f) is the Fourier Transform of y(t); $S_1(f)$ is the Fourier Transform of si(t); and $H_1(f)$ is the Fourier Transform of $h_1(t)$. In such a case, convolution in the time domain becomes a multiplication in the frequency domain.

A logarithm of Equation 4 may be applied so that the multiplication is converted into an addition, resulting in Equation 5:

$$Log\{Y(f)\} = Log\{S_1(f)H_1(f)\} = Log\{S_1(f)\} + Log\{H_1(f)\} \qquad \text{Eq. 5}$$

Equation 5 may then be converted back into the time domain, by an Inverse Fourier Transform (IFT) (e.g., an inverse DFT or inverse FFT), which results in a complex cepstrum $(K(\tau))$ (complex because one can work from the complex spectrum) - the inverse Fourier Transform of the logarithm of the spectrum; Equation 6.

$$K(\tau) = IFT[Log\{S_1(f)\} + Log\{H_1(f)\}] \qquad \text{Eq. 6}$$

where "$\tau$" is a real valued variable known as quefrency, with units measured in seconds. From this, the effects that are convolutive in the time domain become additive in the logarithm of the spectrum, and remain so in the cepstrum.

Consideration of the data from a cepstrum analysis, such as examining the data values of the quefrency, may provide information about the system. For example, by comparing cepstrum data of a system from a prior or known baseline of cepstrum data for the system, the comparison, such as a difference, can be used to recognize differences or similarities in the system that may then be used to implement varying functions or purposes disclosed herein, including determining movement events and body position. The following disclosure can utilize the methodologies of such an analysis, as herein explained, to implement the determination of any movement of the user and/or the body position of the user.

In some implementations, direct spectral methods can be implemented to analyze the acoustic data. Some examples of direct spectral methods include processing discrete Fourier transform (DFT), fast Fourier transform (FFT) (optionally with a sliding window), short time Fourier transform (STFT), wavelet-based analysis, wavelet-based cepstrum calculation, Hilbert-Huang transform (HHT), empirical mode decomposition (EMD), blind source separation (BSS), Kalman filters, or a combination thereof. In some implementations, cepstral coefficients (CCs) such as mel-frequency cepstral coefficients (MFCCs) may be used, for example, by treating the acoustic data analysis in the same way as a speech recognition analysis and using a machine learning / classification system. In other implementations, deep neural networks was be employed using image analysis methods applied to a spectrogram.

In some implementations, the system of the present disclosure includes a flow rate sensor (e.g., the flow rate sensor 134 of FIG. 1) and/or a pressure sensor (e.g., the pressure sensor 132 of FIG. 1). The flow rate sensor 134 can be used to generate flow rate data associated with the user 210 (FIG. 2) of the respiratory therapy device 122 during the sleep session. Examples of flow sensors (such as, for example, the flow rate sensor 134) are described in International Publication No. WO 2012/012835. In some implementations, the flow rate sensor 134 is configured to measure a vent flow (e.g., intentional "leak"), an unintentional leak (e.g., mouth leak and/or mask leak), a patient flow (e.g., air into and/or out of lungs), or a combination thereof.

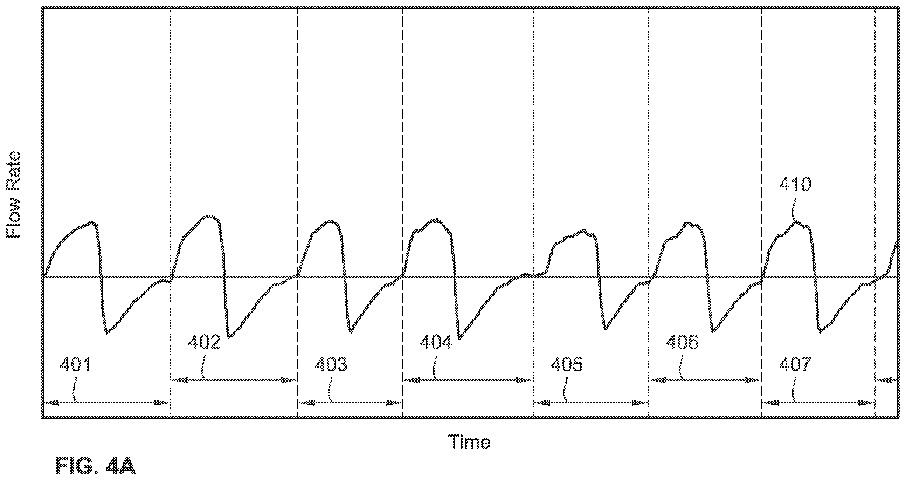

In some implementations, the flow rate sensor and/or a pressure sensor is configured to generate flow and/or a pressure data over a period of therapy time. For example, FIG. 4A illustrates a portion of such flow rate data associated with a user (e.g., the user 210 of FIG. 2) of a respiratory therapy system (e.g., the respiratory therapy system 120 of FIG. 1), according to some implementations of the present disclosure. As shown in FIG. 4A, a plurality of flow rate values measured over about seven full breathing cycles (401-407) is plotted as a continuous curve 410.

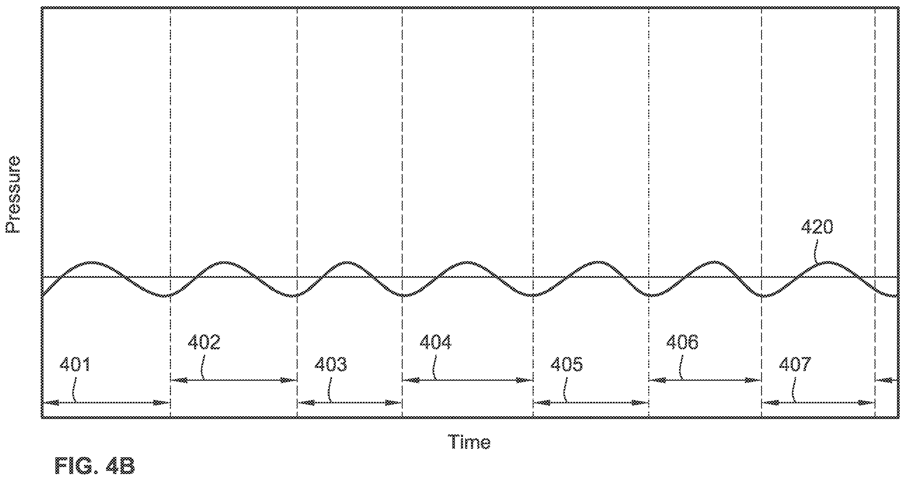

In some implementations, the pressure sensor is configured to generate pressure data over a period of therapy time. For example, FIG. 4B illustrates pressure data associated with a user of a CPAP system, according to some implementations of the present disclosure. The pressure data shown in FIG. 4B was generated over the same period of therapy time as that of FIG. 4A. As shown in FIG. 4B, a plurality of pressure values measured over about seven full breathing cycles (401-407) is plotted as a continuous curve 420. Because a CPAP system is used, the continuous pressure curve of FIG. 4B exhibits a generally sinusoidal pattern with a relatively small amplitude, because the CPAP system attempts to maintain the constant predetermined air pressure for the system during the seven full breathing cycles.

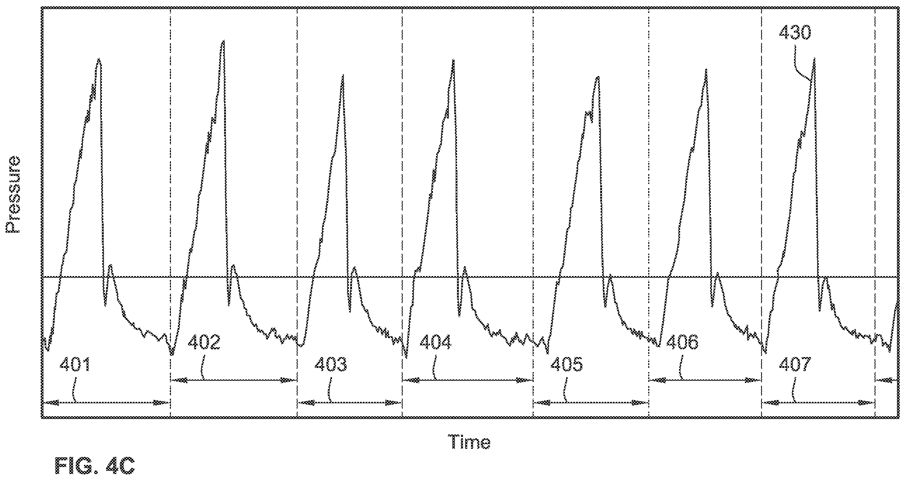

Referring to FIG. 4C, pressure data associated with a user of a respiratory therapy system with an expiratory pressure relief (EPR) module is illustrated, according to some implementations of the present disclosure. The pressure data shown in FIG. 4C was generated over the same period of therapy time as that of FIG. 4A. As shown in FIG. 4C, a plurality of pressure values measured over about seven full breathing cycles (401-407) is plotted as a continuous curve 430. The continuous curve of FIG. 4C is different from that of FIG. 4B, because the EPR (Expository Pressure Relief) module (used for the pressure data in FIG. 4C) can have different settings for an EPR level, which is associated with the difference between a pressure level during inspiration and a reduced pressure level during expiration.

Referring to FIG. 5, a flow diagram for a method 500 for determining movement of a user while using a respiratory therapy system is disclosed. One or more steps of the method 500 can be implemented using any element or aspect of the system 100 (FIGS. 1-2) described herein. The benefits of having the method 500 for movement detection while on therapy include improvements to sleep staging (e.g., classifying wake, light sleep, deep sleep, and/or REM sleep). Sleep staging development so far has been primarily carried out based on the flow signal alone. However, the level of activity while a person is sleeping corresponds with certain sleep states. If a person is awake, they typically move frequently, if a person is in deep sleep or REM sleep, they typically move less or not at all. Therefore, improved movement and/or activity detection can help to better classify sleep states.

At step 510, acoustic data associated with the user of a respiratory therapy system (e.g., the respiratory therapy system 120 of the system 100) is received. For example, in some implementations, the acoustic data is received from a microphone (e.g., the microphone 140 of the system 100) associated with the respiratory therapy system. The microphone is configured to generate the acoustic data based at least in part on detected audio associated with the respiratory therapy system. The acoustic data is reproducible as one or more sounds associated with movement of a component, such as a conduit (e.g., the conduit 126 of the system 100), of the respiratory therapy system. Said movement causes rubbing of at least a portion of the component, e.g. conduit, on one or more surfaces, such as the surface of a bed, bed coverings, the user's body, or the surface of the component (e.g. conduit) itself or another component of the respiratory therapy system.

In some implementations, the microphone is external to the respiratory therapy system. In such implementations, the microphone is coupled to a smart device, a smart phone, a smart speaker, or any combination thereof. In some implementations, the microphone is (i) internal to the respiratory therapy system, (ii) integrated with the respiratory therapy system, or (iii) both (i) and (ii). In some other such implementations, the microphone is at least partially coupled to a component of the respiratory therapy system, such as (i) the conduit 126 of the respiratory therapy device 122 of the respiratory therapy system 120, (ii) a circuit board of the respiratory therapy device 122 of the respiratory therapy system 120, (iii) a connector of the respiratory therapy system 120, (iv) the user interface 124 of the respiratory therapy system 120, or (v) any other component of the respiratory therapy system 120. For example, in some implementations, the microphone can be positioned within a housing of the respiratory therapy device 122.

It is noted that more or fewer microphones can be implemented in the method 500. For example, the method 500 can be implemented using a first microphone and a second microphone, where the first microphone is external to the respiratory therapy system, and the second microphone is (i) internal to the respiratory therapy system, (ii) integrated with the respiratory therapy system, or (iii) both (i) and (ii).

Even though movement data may be determined using the existing flow signal, in some instances, the acoustic signal is more accurate than the flow signal and may be used to determine movement events or to confirm/augment the determination of movement events from the flow signal. For example, some patients may stop breathing and/or breathe less consistently when they move - the flow signal may not accurately reflect such movement, but the acoustic signal would. When both the flow signal and acoustic signal are used to obtain movement data, this can result in a synergy, as well as better detecting movement when a patient stops breathing and/or breathes less consistently. The use of the acoustic signal can confirm and/or add confidence to the movement detected via the flow signal, and *vice versa.*

At step 520, the received acoustic data is analyzed. In some implementations, the analyzing the received acoustic data (step 520) can include one or more of the following steps 522-526. At step 522, a plurality of audio signal amplitudes for a time period (e.g., two seconds, three seconds, four seconds, five seconds, six seconds, seven seconds, eight seconds, nine seconds, ten seconds, etc.) is determined from the received acoustic data. For example, in some implementations, the time period corresponds to a number of breathing cycles, wherein each breathing cycle includes an inhalation portion and an exhalation portion. Additionally, or alternatively, in some implementations, the number of breathing cycles is one breathing cycle, two breathing cycles, three breathing cycles, four breathing cycles, or five breathing cycles.

At step 524, a standard deviation for the time period is calculated based at least in part on the determined plurality of audio signal amplitudes and corresponds to the standard deviation thereof. At step 526, the standard deviation for the time period is compared to a predetermined threshold. In some implementations, the predetermined threshold to be compared with the standard deviation for the time period (step 526) can be determined by one or more of the following steps 530-536. At step 530, a plurality of baseline audio signal amplitudes for a first time period and a plurality of active audio signal amplitudes for a second time period are determined from the received acoustic data. For example, the plurality of baseline audio signal amplitudes for the first time period can correspond to no movement or minor movement (e.g. movement due to normal breathing by the user, movement due to normal heart beating, movement caused by ambient air flow, rapid eye movement during sleep, etc.) associated with the user; and the plurality of active audio signal amplitudes for the second time period can correspond to active movement (e.g. movement due to the user rolling from one body position to another, such as from a supine position to lying on the left side of the body, etc.) associated with the user.

At step 532, a baseline standard deviation is calculated based at least in part on the determined plurality of baseline audio signal amplitudes. At step 534, an active standard deviation is calculated based at least in part on the determined plurality of active audio signal amplitudes. At step 536, the predetermined threshold is determined based at least in part on the calculated baseline standard deviation and the calculated active standard deviation. For example, the predetermined threshold may be determined by averaging (i) the baseline standard deviation and (ii) the active standard deviation. In some implementations, the predetermined threshold can be calculated using historical data and/or real-time data. In some examples, not all recordings would have the same baseline audio signal amplitudes. The predetermined threshold and/or the baseline audio signal amplitudes are selected to provide the best movement detection across different recordings. Additionally, or alternatively, the predetermined threshold and/or the baseline audio signal amplitudes are running and/or adjustable based on the real-time data.

In some implementations, the variation is assessed using a different metric than standard deviation, such as variance. Additionally, or alternatively, in some implementations, the variation is assessed over a different window length, such as one second, two seconds, three seconds, four seconds, five seconds, six seconds, seven seconds, nine seconds, ten seconds, etc. Additionally, or alternatively, in some implementations, the predetermined threshold is applied in another way, such as using a running and/or adjustable threshold.

In some implementations, the analyzing the received acoustic data (step 520) can include a cepstrum analysis, an autocepstrum analysis, an autocorrelation analysis, a spectral analysis, or any combination thereof. In some such implementations, the spectral analysis includes a fast Fourier transform (FFT) (optionally with a sliding window), a spectrogram, a short time Fourier transform (STFT), a wavelet-based analysis, or any combination thereof.

In some implementations, the analyzing the received acoustic data (step 520) can include, at step 540, processing the received acoustic data to identify a plurality of features. In some such implementations, the analyzed acoustic data can include (i) cepstrum data, (ii) spectral data comprising e.g., an acoustic spectral signature, or (iii) both (i) and (ii). For example, in some implementations, the plurality of features includes (i) a change in spectral signature, (ii) a change in frequency, (iii) a change in signal amplitude, (iv) a change in location, or (v) any combination thereof. These one or more changes may include changes that occur as a result of movement, such as the change in one or more features from before movement to after movement. Specifically, for example, the plurality of features may include (i) the change in spectral signature from the spectral signature before movement to the spectral signature after the movement; (ii) the change in frequency from the frequency before movement to the frequency after the movement; (iii) the change in signal amplitude from the signal amplitude before movement to the signal amplitude after the movement, (iv) the change in location from the location before movement to the location after the movement, (v) or any combination thereof. In some implementations, regarding the change in location feature, the movement of the conduit may cause stretching and/or bending, therefore resulting in the change in location associated with the received acoustic data. For example, in some such implementations, if a conduit is stretched, a location of a physical feature in the conduit may change, such as relative to another location of the conduit and/or another physical feature within the conduit.

Additionally, or alternatively, in some implementations, the analyzing the received acoustic data (step 520) can include analyzing the plurality of features using a machine learning model (e.g., Markov, Bayesian, 1D-convolution, logistic regression, or any combination thereof). Such models can be trained on historical body position data and historical acoustic data (e.g., from a user or a population of users).

At step 550, a movement event associated with the user is determined based at least in part on the analysis (e.g., step 520). In some implementations, movement event is associated with (i) a change in user position, (ii) a sleep state, (iii) a change in sleep state, (iv) a sleep stage, (v) a change is sleep stage, or (vi) an occurrence of a respiratory event (e.g., an inhalation, an exhalation, a cough, a gasp, a sneeze, a laugh, a cry, a scream, a snore, an apnea, or any combination thereof). In some implementations, the movement event includes a plurality of movement events.

In some implementations, the determining the movement event associated with the user (step 550) includes determining a movement frequency at step 552. In some implementations, movement frequency is indicative of the sleep stage and/or sleep state of the user, because different sleep stages and/or sleep states have different levels of movement frequencies. In some such implementations, high movement frequency indicates wakefulness and/or restlessness of the user; lower movement frequency indicates the user may be in light sleep; lowest movement frequency and/or zero movement frequency indicates the user may be in deep sleep or REM sleep. In some implementations, the high movement frequency, the lower movement frequency, and/or the lowest movement frequency is relative and/or user specific, as some individuals may move a lot when sleeping, whereas others may move less. In some implementations, clusters of movements may indicate a specific sleep state (e.g., awake). In some implementations, an arbitrary number may be assigned, for example, 0 = no movement and likely asleep, whereas 3 may be associated with some movement and likely awake. In some implementations, the movement frequency may be combined with other features and/or data (e.g., respiration data), which may together indicate and/or confirm the sleep state and/or sleep stage associated with the user. In some implementations, other demographic data (e.g., the age of the user) may be factored in. For example, the movement frequency when the user is asleep generally changes with age. Additionally or alternatively, in some implementations, the movement frequency may be further analyzed to detect sleep movement disorders, such as period leg movement and/or restless leg syndrome. Additionally or alternatively, in some implementations, the movement frequency may be combined with other data that may indicate the sleep state and/or sleep stage of the user (e.g., flow rate data and/or pressure data), and the movement frequency feature is then analyzed to indicate and/or confirm movement.

In some implementations, the audio data may be pre-processed before being analyzed at step 520. For example, in some such implementations, the pre-processing may include: (i) filtering for a certain frequency range (e.g., typically high frequency components are desired for fast events like movement), (ii) downsampling the audio signal to a lower frequency, (iii) a normalization step, or (iv) any combination thereof. Regarding the normalization step, the section values with no movement of the conduit are normalized (e.g., scaled) such that those section values remain equivalent for different sessions of the same user and/or different users. For example, after all the values are normalized based on the no movement section values being equivalent, if a movement does happen, the same threshold (e.g., the predetermined threshold as disclosed herein) is applied to each user to aid in distinguishing movement vs non-movement. As another example, a user can be sleeping in a quiet room versus a loud room. Acoustic data (e.g., the acoustic data received at step 510) received in the quiet room may contain less ambient noise than that in the loud room. Additionally and/or alternatively, in some implementations, the ambient noise may also include background noise generated by components of the respiratory therapy system, such as the microphone and/or the flow generator. As such, the baseline values associated with the analyzed acoustic data from the loud room would be higher than that from the quiet room, and it would then be more difficult to find a threshold that detects the sound indicative of movement of the conduit for that user, at least partially because the acoustic data are received on different scales between those two scenarios. With the normalization step, this difference in scaling can be eliminated, such that the same threshold is used for both scenarios.

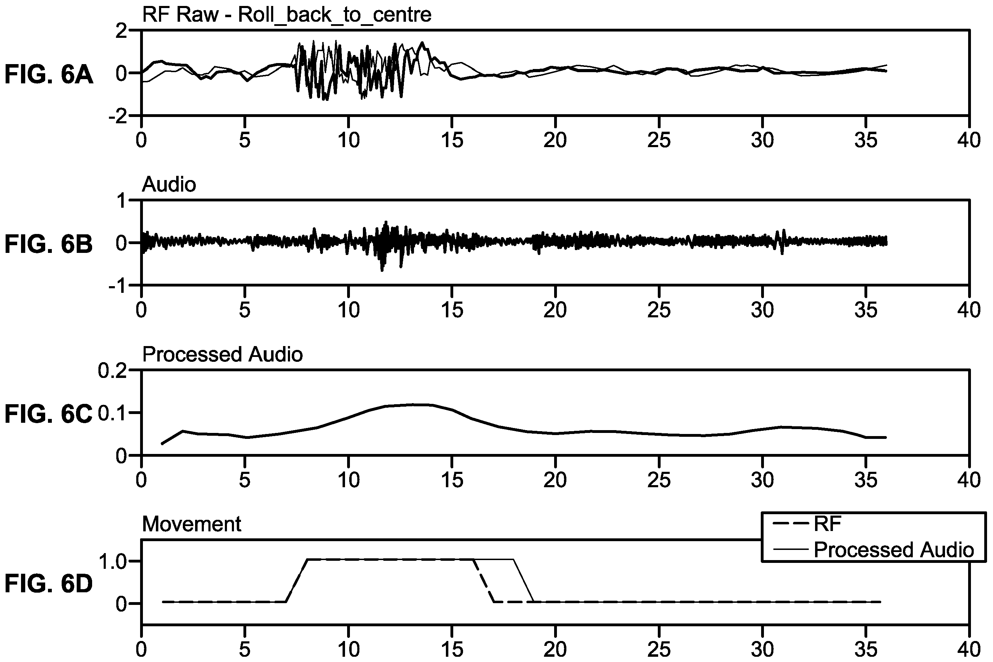

Referring to FIGS. 6A-6D, test data supports that the systems (e.g., the system 100) and methods (e.g., the method 500) disclosed herein can effectively determine movement of a user while using a respiratory therapy system. For example, in some implementations, the method 500 identifies movements by extracting the variation (e.g., standard deviation) in the audio signal over a time window, e.g. a window of 5 seconds (or approximately one or two breaths). Thus, sliding and/or shifting this window in time gives the running standard deviation of the audio signal. Then, stable breathing with no or minor movement acts as a baseline with a low and consistent standard deviation, while active movements result in peaks in this metric. The increase in this metric is due to impulses (due to, for example, conduit movements), and more varied and inconsistent breathing patterns during physical movement.

FIG. 6A illustrates RF (e.g., RADAR) data measured during a therapy session as indicative of movement of a subject, according to some implementations of the present disclosure. FIG. 6B illustrates audio data measured during the therapy session of FIG. 6A. FIG. 6C illustrates processed audio data calculated using the audio data of FIG. 6B. FIG. 6D illustrates movement activity data calculated using the processed audio data of FIG. 6C. Thus, FIGS. 6B-6C show a movement analysis versus a validated reference (FIG. 6A).

In this example, to determine the processed audio in FIG. 6C, the standard deviation is calculated for a first audio sample (consisting of a period of eight seconds from 0s to 8s), then the sliding window shifts to the next sample, which is 0.1 second forward in time (consisting of a period of eight seconds from 0.1s to 8.1s). Any constraints of the system from buffering the samples can be alleviated by down sampling first.

As shown in FIG. 6B, the audio signal between 10 and 15 seconds shows some disturbance. In contrast, before 10 seconds and after 15 seconds, the audio signal shows stable breathing with no or minor movement. As shown in FIG. 6C, the processed audio metric picks up this variation moving from a baseline of about 0.05 to > 0.1 in standard deviation during this movement period.

In FIGS. 6A-6D, the X-axis is the time. In FIG. 6A, the Y-axis is the RF sensor output in volts (V). The RF sensor has two channels - I (in-phase) and Q (quadrature), each of which is plotted in FIG. 6A. In FIG. 6B, the Y-axis is the amplitude of the audio signal that is recorded during the same period that the RF signal in FIG. 6A is recorded. In FIG. 6C, the Y-axis is standard deviation of the processed audio signal from FIG. 6B. In FIG. 6D, the Y-axis is 1 or 0, where 1 indicates movement and 0 indicates no movement, where movement is defined as where the standard deviation (FIG. 6C) being greater than 0.075. Specifically, FIG. 6D is plotted such that movement is true (=1) if the processed audio signal (FIG. 6C) exceeds the theoretical threshold of standard deviation (i.e., 0.075 in this example), and movement is false (=0) if the processed audio signal (FIG. 6C) does not exceed the same theoretical threshold of standard deviation. In other words, by setting a predetermined threshold for the processed audio metric (e.g. at 0. 075 as in the present example of FIG. 6C), every sample that the processed audio metric is above 0.075 is marked as a movement event (e.g., movement can be either 0 - indicative of no or little movement, or 1 - indicative of movement event). Even though this example shows 0.075 as the predetermined threshold, the predetermined threshold using this processed audio metric can be other values between about 0.05 and about 0.1. In some implementations, the predetermined threshold can be any value selected that distinguishes movement.

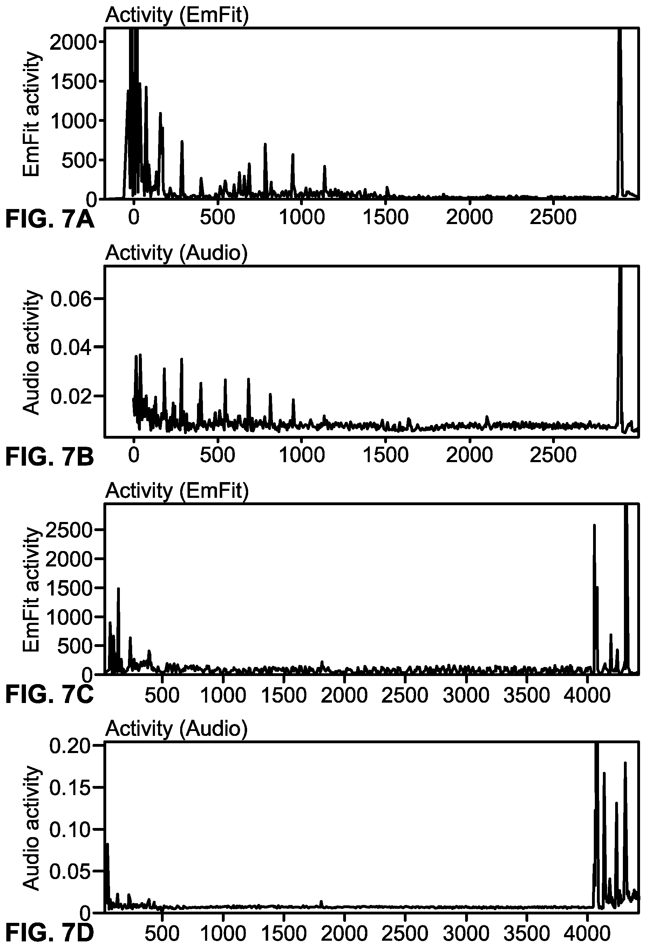

Referring now to FIGS. 7A-7D, additional test data further supports that the systems (e.g., the system 100) and methods (e.g., the method 500) disclosed herein can effectively determine movement of a user while using a respiratory therapy system. FIG. 7A illustrates EMFIT activity data measured during a first therapy session, according to some implementations of the present disclosure. FIG. 7B illustrates processed audio data derived from audio signals detected by a microphone within the conduit of the respiratory therapy system, which is measured during the first therapy session of FIG. 7A, according to some implementations of the present disclosure. FIG. 7C illustrates EMFIT activity data measured during a second therapy session, according to some implementations of the present disclosure. FIG. 7D illustrates audio data measured during the second therapy session of FIG. 7C, according to some implementations of the present disclosure. This activity metric versus the EMFIT device shows good alignment (calculated by comparing variations between each breath, and/or the average at every fixed eight seconds which captures about two breaths).

In this example of test data illustrated in FIGS. 7A-7D, the microphone is positioned inside the conduit, near the end of the conduit that connects to the respiratory device (comprising the flow generator). Positioning the microphone within the respiratory system, e.g. within the conduit, respiratory device, or user interface, results in the microphone being particularly sensitive at picking up sounds caused by movement of components of the respiratory system, in particular the conduit. In some such implementations, the sound detected by the microphone may be caused by movement of the conduit on/against the bedding. Additionally or alternatively, in some other implementations, the sound detected by the microphone may be caused, or also caused, by another source without the conduit contacting (e.g., rubbing against) another surface, such as movement of the conduit itself causing a vibration within the conduit. In some such implementations, a microphone may be optionally positioned elsewhere, e.g., outside the respiratory therapy system. This placement outside of the respiratory therapy system may improve detection of other sounds associated with the movement of the user, e.g., creak from bed and/or rustle of bedding as user rolls over, etc.

Referring to FIG. 8, a flow diagram for a method 800 for determining a body position of a user during a respiratory therapy session is disclosed. One or more steps of the method 800 can be implemented using any element or aspect of the system 100 (FIGS. 1-2) described herein. At step 810, flow rate data (e.g., generated by flow rate sensor 134) associated with the user of a respiratory therapy system (e.g., the respiratory therapy system 120 of the system 100) is received. At step 820, the received flow rate data is analyzed to determine one or more features. At step 830, the body position of the user is determined based at least in part on the determined one or more features. For example, clusters of respiratory events are more likely when the user is lying on the back. Relative increase in frequency of respiratory events and/or clusters of respiratory events, can also indicate that the user is lying on the back. In some implementations, if the analyzed flow rate data is indicative of a cluster of apneas, a movement, and less apneas, then method 800 provides that the body position of the user is likely changed from supine to non-supine.

Additionally, or alternatively, in some implementations, pressure data (e.g., generated by pressure sensor 132) is received and analyzed in the same, or similar manner as described herein. Thus, although FIG. 8 illustrates an example using flow rate data, it is contemplated that flow rate data, pressure data, or both can be used for determining the body position of the user using the method 800.

The body positions can include: (i) Left-Side, (ii) Right-Side, (iii) Prone (on stomach), and (iv) Supine (on back). The Left-Side body position during sleep can reduce issues such as insomnia and/or gastroesophageal reflux disease (GERD), which can both contribute negatively to sleep apnea. The user may experience the best quality sleep - better blood flow, and reduced resistance to breathing - in the Left-Side body position during sleep. Unfortunately, the Left-Side body position may also result in a higher mask leak than other body positions, e.g. Supine, depending on mask type and/or if the mask is incorrectly fitted. The user's heart rate should be monitored in cases of comorbid Congestive Heart Failure (CHF), as the Left-Side body position may not be a good position. The Right-Side body position during sleep can be an alternative to Left-Side body position, and carries the same, or similar, advantages and disadvantages as discussed herein with regard to the Left-Side body position.

The Prone (on stomach) body position can reduce obstructive respiratory events, as the user's tongue tends to roll forward rather than back as can occur in an obstructive respiratory event. However, the user's mouth may be occluded by pillow bedding. In addition, in some instances, the Prone body position may cause mask leaks and/or discomfort with some mask types. The Supine (on back) body position may provide for the best mask seal. However, the Supine body position requires higher pressures, as the user's tongue tends to roll back. Thus, for users with positional apnea, the Supine body position tends to be the worst position.

In some implementations, the determining the body position of the user includes, at step 832, comparing the determined one or more features from acoustic and/or flow rate data to a plurality of historical features associated with a plurality of historical body positions. For example, in some implementations, at step 840, historical flow rate data associated with the user of the respiratory therapy system during a respiratory therapy session is received.

At step 850, a plurality of movement events associated with the user is determined using any suitable means such as, for example, an RF sensor, EMFIT device, pressure sensitive mattress, etc. Based at least in part on the determined plurality of movement events, at optional step 852, the respiratory therapy session is divided into a plurality of respiratory therapy sections. At optional step 854, the received historical flow rate data for each of the plurality of respiratory therapy sections is analyzed to extract the plurality of historical features. Alternatively, the plurality of historical features is extracted from the historical flow rate data without having divided the respiratory therapy session into sections. Based at least in part on the analysis, at step 856, a historical body position of the plurality of historical body positions is determined, optionally for each of the plurality of respiratory therapy sections, and correlated with one or more of the plurality of historical features extracted from the historical flow rate data.

In some implementations, steps 840-856 may include a reference dataset where (i) it is known what body position a user is in for a given timeframe; (ii) features of the flow and/or pressure signals are extracted, which are correlated with the body position of the user in the given timeframe; and (iii) a machine-learning model is then trained based on these body position data and flow and/or pressure signal features. Then, for the newly collected flow and/or pressure signal data, which body position the user is in can be determined by (i) extracting the features that aid in the determining the body position of the user, and feeding these features into the trained machine-learning model, or (ii) feeding the flow and/or pressure signal data directly into the trained machine-learning model; and (iii) receiving the body position returned by the machine-learning model. In some implementations, the machine-learning model derived in steps 840-856 using the reference dataset may then be used in step 820 and/or step 830 of the method 800.

In some implementations, the features (e.g., features associated with acoustic data, flow rate data, and/or pressure data) are extracted on an epoch-to-epoch basis. For example, a sleep session (during which the acoustic data, flow rate data, and/or pressure data is generated) is divided into a plurality of individual segments referred to as epochs. The features are extracted for each epoch, and may be analyzed independently or relatively to one another throughout the sleep session. Additionally or alternatively, in some implementations, physiological data can also be generated, which may include a respiration signal representative of the user's respiration. Generally, the respiration signal is a measure of the amplitude of the user's respiration. Further additionally or alternatively, in some implementations, temporal data can also be generated by system 100 (e.g., data related to the time duration of the sleep session, data related to which epoch of the sleep session is the current epoch, etc.).

The received data may be analyzed to determine, for every individual epoch in the sleep session, which body position that the user is in for that epoch. Generally, the data is analyzed in real-time, and thus every time an epoch ends, the data from that epoch is analyzed to determine which body position the user was in during that epoch. For example, in some implementations, each epoch may last for 30 seconds, and thus the data in this example is analyzed in 30-second chunks. In other implementations, however, different lengths for the epochs can be used. For example, the length of the epochs could be 1 second, 5 seconds, 10 seconds, 20 seconds, 1 minute, 2 minutes, 5 minutes, or 10 minutes. The length of the epochs can also be set to correspond to a specific number of breaths, such as 1 breath, 2 breaths, 5 breaths, etc. The length can be determined based on the user's average respiration rate, or an average respiration rate for a population to which the user belongs.

As another example, analysis on an epoch-to-epoch basis may be implemented as follows. First, data associated with a sleep session that has a plurality of epochs is received. In some implementations, the data includes flow rate data representative of the flow of pressurized air from the respiratory therapy device 122 to the user interface 124 via the conduit 126. The flow rate data is analyzed to identify features associated with the current epoch. As disclosed herein, the sleep session can be divided into a plurality of epochs. The epochs can be any suitable length, such as 30 seconds long or for the length of one breath.

Any flow rate data representative of the flow of pressurized air during the epoch is analyzed. Additionally, in some implementations, any respiration data representative of the user's respiration rate during the epoch is also analyzed, which may be determined based on the flow rate data and/or other data generated using one or more sensors 130. A number of different features can be identified. In some implementations, the features include one or more features that are associated with the flow of the pressurized air, such as (i) a maximum flow value across the current epoch and one or more prior epochs, (ii) a flow skew of the current epoch, (iii) a median flow skew across the current epoch and one or more prior epochs, (iv) a standard deviation of flow values for the current epoch, (v) a standard deviation of the standard deviation of flow values for the current epoch and one or more prior epochs, (vi) standard deviation of the flow volume for the current epoch and one or more of the prior epochs (vii) a time ratio of inspiration to expiration for the current epoch, (viii) a ratio of inspiration volume to expiration volume for the current epoch, or (ix) any combination of (i)-(viii).

In some implementations, the features include one or more features that are associated with the respiration rate of the user, such as the average respiration rate across the current epoch, a standard deviation of the respiration rate across the current epoch and one or more prior epochs, or both. In some implementations, the features can include at least one feature associated with a temporal property of the current epoch. Generally, the temporal property is some measurement of where the epoch is located within the sleep session. For example, the temporal property can be the number of the current epoch within the current sleep session or the $n^{th}$ root of the number of the current epoch within the current sleep session. In some implementations, n=20, and the temporal property is the $20^{th}$ root of the number of the current epoch within the current sleep session.

The extracted features from flow rate data (and/or the respiration data) are then analyzed to identify movements associated with the user during the current epoch. This analysis can indicate if the user has moved and/or the movement frequency during the current epoch. After features from the current epoch have been extracted and movements occurring during the current epoch have been identified, the plurality of body position probabilities can be adjusted, based on the movements occurring during the current epoch, movements occurring during the prior epoch, or the determined body position of the prior epoch.

In some examples, by detecting when movement events occur, the sleep session can be divided into different sections. In an illustrative example, 0 represents no movement or little movement (e.g., movement due to respiration, slight movements of the body), and X represents a movement event (e.g., movements of the body due to movements of limbs and/or movement from one lying position to another, such as from supine to left side). A sleep session may be expressed as:

0000000X000000000000X0X0X000000000000

Such sections can then be analyzed separately. Extracting features of the flow and/or pressure signals within a section can help understand what position the user is in. For example, in some implementations, features relating to respiratory mechanics of each position can help to identify the body position (e.g., respiratory effort, for example, may be greater/lesser in supine vs. lateral positions). If it is more difficult to breathe in one body position, the user may breathe a lower volume of air. By extracting features from the flow signal (such as flow signal amplitude, tidal volume and/or minute ventilation), and comparing to features prior to the movement, the method 500 can determine whether the user has moved from one position to another, and/or in which body positions the user may be. For example, in some implementations, features such as flow signal amplitude, tidal volume and/or minute ventilation can decrease when a user moves from supine to lateral position, and lateral to prone position

In some implementations, the flow signal in real time is analyzed in order to detect a change a body position and then classifies the new position. The method 800 then allows the system 100 to change the pressure of the respiratory therapy system 120 to best provide therapy to the user based on the user's body position. The method 800 may employ processing so as to not change settings, if the user rolls over and then back to the same position in a short period of time (e.g., within about 5 seconds), and/or if the users moves in bed without changing position.

Other examples of the flow and/or pressure data features that may be used for determining the body position include signal amplitudes (e.g., peak inspiration/expiration), morphology of the breathing waveform, breathing frequency, ratio of inhalation to exhalation durations, analysis of the breathing signal envelopes over time, or a combination thereof. In some implementations, these features are in part user specific, where same trends may present for each user, but the values may differ between different users.

Leak information, such as mask leak or mouth leak information, may be derived from flow and/or pressure signals (and/or acoustic signals) and may also be used to help infer changes in body position. For example, if there is a sudden change in leak, the sudden change may imply the mask has shifted slightly on the user's face and/or the user has begun (or ceased) breathing through their mouth, due to a positional change. Other information such as determination of snore from flow, pressure, and/or acoustic data, may also be indicative of a higher likelihood of the user sleeping in the supine position.

In some implementations, signal processing techniques such as frequency domain analysis, auto-correlation, filtering, finding fiducial points, and/or calculating a derivative of the signal can be applied as preprocessing steps prior to extracting features. Any features extracted can then be input into any of a variety of statistical or machine learning models such as Markov, Bayesian, 1D-convolution, logistic regression, and/or any other such classifier to determine the current position as supine, lateral, or prone. Such models can be trained on historical body position data and historical flow and/or pressure data/extracted features (e.g., from a user or a population of users).

In some implementations, for example, at step 860, the body position of the user is labeled with a time stamp. In some implementations, for example, at step 862, one or more parameters (e.g., motor speed, pressure, flow rate, or any combination thereof) of the respiratory therapy session are modified based at least in part on the determined body position for the user. Additionally, or alternatively, in some implementations, at step 864, an alarm, a notification, or an instruction is generated based at least in part on the determined body position for the user. Additionally, or alternatively, in some implementations, movement of the user is determined based at least in part on the received acoustic data (step 810) according to the method 500 disclosed herein.

In some implementations, secondary data (e.g., received from another sensor such as one or more sensors 130 of the system 100) in addition to the flow rate data may be used to determine the one or more features (step 820). For example, in some such implementations, at step 870, acoustic data associated with the user of the respiratory therapy system (e.g., generated by microphone 140) is received. In some such implementations, the body position of the user is further determined based at least in part on the received acoustic data associated with the user of the respiratory therapy system.

The method 800 allows for detection of body position and/or changes to body position of a user during sleep. As disclosed herein, positional changes can be detected by tracking flow features following large movements (e.g., as detected by the microphone of the respiratory therapy system). In some implementations, those features and/or characteristics in the flow signal are variable in relation to sleep position. Because therapy requirements are also variable with sleep position, the therapy can be customized accordingly based at least in part on determined positional changes throughout the therapy session.

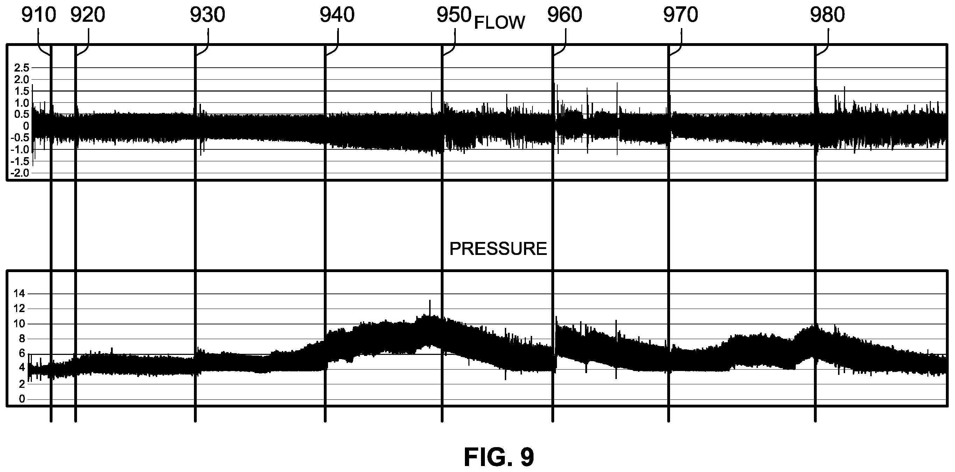

FIG. 9A illustrates a real-time flow signal and a real-time pressure signal measured simultaneously during a therapy session, according to some implementations of the present disclosure. The method 800 (FIG. 8) can process the flow signal in real time, and calculate long term baseline trends of the signal, peak to trough amplitudes of the signal, breathing rate, and/or breath shapes in order to determine the user's body position (and thus to detect a change in position) and to classify the new position. This can be coupled to the respiratory therapy system, to monitor apneas and hypopneas.

In some implementations, the body position is estimated from flow-based and/or pressure-based features at regular intervals. The change in body position is then determined based on whether there is a change in the estimated body position. Additionally or alternatively, in some implementations, only the flow-based and/or pressure-based features during stable time periods are used to determine the body position, so that any randomness in the flow signal and/or the pressure signal (e.g., due to possible limb (but not body position) movements, coughs, respiratory events) do not impact the estimation of the body position. In some other implementations, any randomness in the flow signal and/or the pressure signal may also indicate a change to a new body position, as such the flow-based and/or pressure-based features during these unstable time periods may be analyzed under the assumption that the relative position is new. Additionally or alternatively, in some implementations, frequency of respiratory events is indicative of the body position. Further additionally or alternatively, in some implementations, an algorithm is trained, such as described herein, to classify a body position based on a selection of flow-based and/or pressure-based data or extracted features.

In some implementations, the flow features can include (i) baseline and peak-peak of flow signal over many breaths (long timescale); (ii) variability of breathing rate, which may include an interference to breath metrics (real time), analysis over longer timescales (e.g., 30 seconds, 60 seconds, 90 seconds, 5 minutes), the stability over time (related to the variability), and/or the standard deviation of breathing rate; (iii) the depth of respiration (e.g., shallow, deep, etc.) and/or relative flow signal amplitude of adjacent breaths, which may include the mean or average value of the breathing rate, and/or the trimmed mean (e.g., at 10%) to reject outliers; (iv) wake or asleep (e.g., the detected sleep state of the user), where surges (e.g., sudden accelerations or decelerations) in breathing rate seen during quiet periods and during REM sleep; (v) skewness of breath shape metrics; (vi) kurtosis of breath shape metrics; (vii) characteristic patterns in the spectrogram; (vii) relative percentage of REM and deep sleep elapsed to date.

As shown in FIG. 9, eight suspected body position changes based on baseline amplitude of flow signal is illustrated at 910, 920, 930, 940, 950, 960, 970, and 980. The body position changes may be determined using the method 800 (FIG. 8) as disclosed herein. The pressure signal below the flow signal shows lagging behavior of APAP in adjusting pressure.

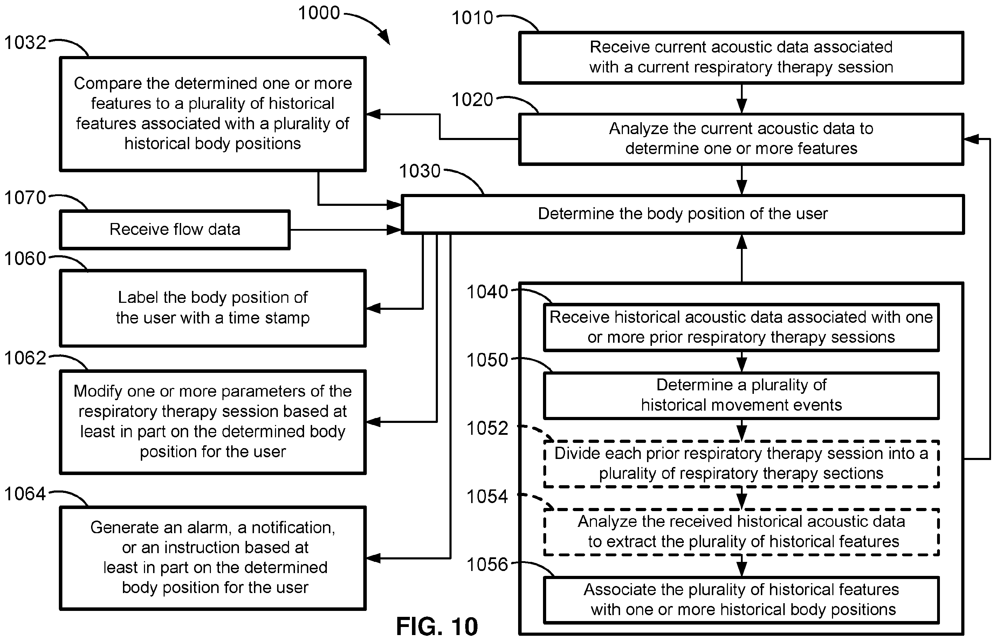

One of the benefits of being able to detect movement (e.g., the method 500) is to detect a change in body position. Body position (e.g., supine, prone, lateral) changes throughout the night. In order to have a change in body position, there must have also been a movement. Referring to FIG. 10, a flow diagram for a method 1000 for determining a body position of a user during a respiratory therapy session is disclosed. One or more steps of the method 1000 can be implemented using any element or aspect of the system 100 (FIGS. 1-2) and/or the method 500 (FIG. 5) described herein.

At step 1010, acoustic data associated with the user of a respiratory therapy system (e.g., the respiratory therapy system 120 of the system 100) is received. For example, in some implementations, the acoustic data may be received from a microphone (e.g., the microphone 140 of the system 100) associated with the respiratory therapy system. In some such implementations, the microphone is configured to generate the acoustic data based at least in part on detected audio associated with the respiratory therapy system. For example, in some implementations, the acoustic data is reproducible as one or more sounds associated with movement of a conduit (e.g., the conduit 126 of the system 100) of the respiratory therapy system. Additionally, and/or optionally, in some implementations, said movement causes rubbing of at least a portion of the conduit on one or more surfaces.

At step 1020, the received acoustic data is analyzed to determine one or more features. At step 1030, the body position of the user is determined based at least in part on the determined one or more features. In some implementations, the acoustic data received from the microphone at step 1010 may include echo data (e.g., based on acoustic reflections), audio data (e.g., based on passive audio signals), or both. For example, the method 1000 may include one or more of the following steps at steps 1010, 1020, and/or 1030: (i) acoustic data associated with acoustic reflections of a sound signal emitted into the conduit and/or mask is received at step 1010 (or, alternatively, acoustic data associated with sound of the conduit rubbing against itself or another surface (e.g., surface of user, surface of bedding, etc.) is received at step 1010); (ii) a ceptsrum or spectral analysis of the acoustic data is performed at step 1020, which may include observing the effective length of the conduit or sudden changes to cepstrum or spectrum as conduit/mask/user moves (or, alternatively, the noise generated by the act of the conduit rubbing against itself or another surface generates a characteristic resonant sound that becomes the noise source for the analysis (e.g., cepstrum or spectral analysis) performed at step 1020).

In some implementations, the determining the body position of the user includes, at step 1032, comparing the determined one or more features to a plurality of historical features associated with a plurality of historical body positions. For example, in some implementations, at step 1040, historical acoustic data associated with the user of the respiratory therapy system during a respiratory therapy session is received.

Based at least in part on the received historical acoustic data, at step 1050, a plurality of movement events associated with the user is determined. Based at least in part on the determined plurality of movement events, at optional step 1052, the respiratory therapy session is divided into a plurality of respiratory therapy sections. At optional step 1054, the received historical acoustic data for each of the plurality of respiratory therapy sections is analyzed to extract the plurality of historical features. Based at least in part on the analysis, at step 1056, a historical body position of the plurality of historical body positions is determined, optionally for each of the plurality of respiratory therapy sections.

In some implementations, steps 1040-1056 may include a reference dataset where (i) it is known what body position a user is in for a given timeframe; (ii) features of the audio signals and/or processed audio data are extracted, which are correlated with the body position of the user in the given timeframe; and (iii) a machine-learning model is then trained based on these body position data and audio signal / processed audio data features. Then, for the newly collected audio signal / processed audio data, which body position the user is in can be determined by (i) extracting the features that aid in the determining the body position of the user; (ii) feeding these features into the trained machine-learning model; and (iii) receiving the body position returned by the machine-learning model. In some implementations, the machine-learning model derived in steps 1040-1056 using the reference dataset may then be used in step 1020 and/or step 1030 of the method 1000.

In some implementations, for example, at step 1060, the body position of the user is labeled with a time stamp. In some implementations, for example, at step 1062, one or more parameters (e.g., motor speed, pressure, flow rate, or any combination thereof) of the respiratory therapy session are modified based at least in part on the determined body position for the user. Additionally, or alternatively, in some implementations, at step 1064, an alarm, a notification, or an instruction is generated based at least in part on the determined body position for the user. Additionally, or alternatively, in some implementations, movement of the user is determined based at least in part on the received acoustic data (step 1010) according to the method 500 disclosed herein.

In some implementations, secondary data (e.g., received from another sensor such as one or more sensors 130 of the system 100) in addition to the acoustic data may be used to determine the one or more features (step 1020). For example, in some such implementations, at step 1070, flow rate data associated with the user of the respiratory therapy system is received. In some such implementations, the body position of the user is further determined based at least in part on the received flow rate data associated with the user of the respiratory therapy system.

Being able to detect a change in body position (e.g., methods 800 and 1000) provides multiple benefits for a user during therapy. Similar to differences in respiratory mechanics due to body position, likelihood of obstructive respiratory events (e.g., apneas, hypopneas, etc.) differs with body position. For example, as obstructive sleep apnea (OSA) is due to an obstruction in the respiratory path, when a user is lying in a supine position, there is a greater mass pushing down on the user's airway. If there is a tendency for OSA events when the user is in a particular body position, a greater pressure setting may be required to keep the airway open in that body position. Conversely, if the user moves to lateral position, the user should not need the same level of therapy pressure. Therefore, knowing the body position could allow the system 100 to tailor therapy pressure to that body position, and optionally also to sleep state (e.g., obstructive respiratory events are more likely in REM and therefore may adjust therapy pressure if, for example, supine body position during REM sleep is detected), and in turn increase patient comfort and/or therapy efficacy.

*Example for Therapy Adjustment Based on Detected Body Position:*

- *Patient is in lateral position; therapy pressure is $10 cmH_2O$*

- *Patient moves*
- *Patient is now in supine position and therapy pressure ramps up to 12cmH$_2$O*

In addition, the user's body position during therapy may be used for the system 100 to recommend alternative solutions to help the user manage their condition. For example, if it is determined that the user suffers primarily from positional apnea, the system 100 may recommend specific pillows targeted at reducing sleep in supine position in order to lower their AHI, which improves patient comfort and sleep quality. Furthermore, body position could also be tracked over time to show the efficacy of such solutions in reducing AHI.

Generally, the methods 600, 800, 1000 can be implemented using a system having a control system with one or more processors, and a memory storing machine readable instructions. The controls system can be coupled to the memory; the methods 600, 800, 1000 can be implemented when the machine readable instructions are executed by at least one of the processors of the control system. The methods 600, 800, 1000 can also be implemented using a computer program product (such as a non-transitory computer readable medium) comprising instructions that when executed by a computer, cause the computer to carry out the steps of the methods 600, 800, 1000.

While the system 100 and the methods 600, 800, 1000 have been described herein with reference to a single user, more generally, the system 100 and the methods 600, 800, 1000 can be used with a plurality of users simultaneously (e.g., two users, five users, 10 users, 20 users, etc.). For example, the system 100 and the methods 600, 800, 1000 can be used in a cloud monitoring setting.

Additionally, or alternatively, in some implementations, the system 100 and/or the methods 600, 800, 1000 can be used to monitor one or more patients while using one or more respiratory therapy systems (e.g., a respiratory therapy system described herein). For example, in some such implementations, a notification associated with movement event(s) and/or body position(s) associated with the one or more patients can be sent to a monitoring device or personnel. The movement event(s) and/or body position(s) can be determined using one or more steps of the methods 600, 800, and/or 1000. Additionally, or alternatively, in some implementations, the movement event(s) and/or body position(s) are simply being recorded.

**Claims**

1. A system (100) for determining movement of a user while using a respiratory therapy system, the system comprising:

   the respiratory therapy system including a conduit (126), a user interface (124), and a respiratory therapy device;
   a microphone (140) positioned within the conduit, the user interface, or the respiratory therapy device of the respiratory therapy system and configured to generate acoustic data;
   a control system (110) including one or more processors (112); and
   a memory (114) having stored thereon machine-readable instructions, wherein the machine-readable instructions, when executed by the one or more processors, cause the system to:

   receive acoustic data associated with the user of the respiratory therapy system that is generated via the microphone, wherein the acoustic data associated with the user is reproducible as one or more sounds associated with movement of a component of the respiratory therapy system, wherein the one or more sounds associated with movement of the component of the respiratory therapy system are caused by a movement of the component of the respiratory therapy system rubbing on one or more surfaces;
   analyze the acoustic data; and
   determine, based on the acoustic data associated with the user that is reproducible as one or more sounds associated with movement of a component of the respiratory therapy system caused by movement of the component of the respiratory therapy system rubbing on one or more surfaces, a movement event associated with the user.

2. The system of claim 1, wherein the respiratory therapy system comprises the control system, the memory, and the microphone.

3. The system of claim 1, wherein the component of the respiratory therapy system is a conduit of the respiratory therapy system.

4. The system of any one of claims 1 to 3, wherein the microphone is external to the respiratory therapy system.

5. The system of any one of claims 1 to 4, wherein the microphone is (i) internal to the respiratory therapy system, (ii) integrated with the respiratory therapy system, or (iii) both (i) and (ii).

6. The system of any one of claims 1 to 5, wherein the movement event is associated with (i) a change in user position, (ii) a sleep state, (iii) a change in sleep state, (iv) a sleep stage, (v) a change is sleep stage, (vi) an occurrence of a respiratory event, or any combination of (i) to (vi).

7. The system of any one of claims 1 to 6, wherein the microphone includes a first microphone and a second microphone, the first microphone being external to the respiratory therapy system, and the second microphone being (i) internal to the respiratory therapy system, (ii) integrated with the respiratory therapy system, or (iii) both (i) and (ii).

8. The method of any one of claims 1 to 7, wherein the movement event includes a plurality of movement events, wherein, optionally, the determining the movement event associated with the user includes determining a movement frequency.

9. The system of any one of claims 6 to 8, wherein the respiratory event includes an inhalation, an exhalation, a cough, a gasp, a sneeze, a laugh, a cry, a scream, a snore, an apnea, or any combination thereof.

10. The system of any one of claims 1 to 9, wherein the machine-readable instructions, when executed by the one or more processors, further cause the system to:

   determine, from the acoustic data, a plurality of audio signal amplitudes for a time period;
   based at least in part on the determined plurality of audio signal amplitudes, calculate a standard deviation for the time period; and
   compare the standard deviation for the time period to a predetermined threshold;

11. The system of claim 10, wherein the machine-readable instructions, when executed by the one or more processors, further cause the system to:

   determine, from the acoustic data, a plurality of baseline audio signal amplitudes for a first time period and a plurality of active audio signal amplitudes for a second time period;
   based at least in part on the determined plurality of baseline audio signal amplitudes, calculate a baseline amplitude standard deviation;
   based at least in part on the determined plurality of active audio signal amplitudes, calculate an active amplitude standard deviation; and
   based at least in part on the calculated baseline amplitude standard deviation and the calculated active amplitude standard deviation, determine the predetermined threshold;
   wherein, optionally, the plurality of baseline audio signal amplitudes for the first time period corresponds to no movement or minor movement associated with the user, and/or
   wherein the plurality of active audio signal amplitudes for the second time period corresponds to active movement associated with the user, and/or
   wherein the predetermined threshold is determined by averaging (i) the baseline standard deviation and (ii) the active standard deviation.

12. The system of any one of claims 10 to 11, wherein the time period is two seconds, three seconds, four seconds, five seconds, six seconds, seven seconds, eight seconds, nine seconds, or ten seconds, and/or wherein the time period corresponds to a number of breathing cycles, wherein each breathing cycle includes an inhalation portion and an exhalation portion, wherein, optionally, the number of breathing cycles is one breathing cycle, two breathing cycles, three breathing cycles, four breathing cycles, or five breathing cycles.

13. The system of any one of claims 1 to 12, wherein the analyzing the acoustic data includes a cepstrum analysis, an autocepstrum analysis, an autocorrelation analysis, a spectral analysis, or any combination thereof, wherein, optionally, the spectral analysis includes a fast Fourier transform (FFT), a spectrogram, a short time Fourier transform (STFT), a wavelet-based analysis, or any combination thereof.

14. The system of any one of claims 1 to 13, wherein the analyzing the acoustic data includes processing the received acoustic data to identify a plurality of features associated with movements events of the user, wherein, optionally, the plurality of features includes (i) a change in spectral signature, (ii) a change in frequency, (iii) a change in signal amplitude, or (iv) any combination thereof.

15. The system of 6, wherein the microphone is coupled to a component of the respiratory therapy system, the component of the respiratory therapy system being selected from (i) a conduit of a respiratory therapy device of the respiratory therapy system, (ii) a circuit board of the respiratory therapy device of the respiratory therapy system, (iii) a connector of the respiratory therapy system, (iv) a user interface of the respiratory therapy system, or (v) any other component of the respiratory therapy system.

**Patentansprüche**

1. System (100) zum Bestimmen einer Bewegung eines Benutzers während der Nutzung eines Atemtherapiesystems, wobei das System Folgendes umfasst:

   das Atemtherapiesystem, das eine Leitung (126), eine Benutzerschnittstelle (124) und eine Atemtherapievorrichtung einschließt;
   ein Mikrofon (140), das innerhalb der Leitung, der Benutzerschnittstelle oder der Atemtherapievorrichtung angeordnet ist und so konfiguriert ist, dass es akustische Daten erzeugt;
   ein Steuerungssystem (110), das einen oder mehrere Prozessoren (112) einschließt; und
   einen Speicher (114), der maschinenlesbare Anweisungen darauf gespeichert aufweist, wobei die maschinenlesbaren Anweisungen, wenn sie von dem einem oder den mehreren Prozessoren ausgeführt werden, das System veranlassen zum:

   Empfangen von akustischen Daten, die dem Benutzer des Atemtherapiesystems zugeordnet sind und über das Mikrofon erzeugt werden, wobei die dem Benutzer zugeordneten akustischen Daten als ein oder mehrere Geräusche wiedergegeben werden können, die einer Bewegung einer Komponente des Atemtherapiesystems zugeordnet sind, wobei das eine oder die mehreren Geräusche, die einer Bewegung der Komponente des Atemtherapiesystems zugeordnet sind, durch eine Bewegung der Komponente des Atemtherapiesystems verursacht werden, die an einer oder mehreren Oberflächen reibt;
   Analysieren der akustischen Daten; und
   Bestimmen, auf Grundlage der dem Benutzer zugeordneten akustischen Daten, die als ein oder mehrere Geräusche wiedergegeben werden können, die einer Bewegung einer Komponente des Atemtherapiesystems zugeordnet sind, und die durch die Reibung der Komponente des Atemtherapiesystems an einer oder mehreren Oberflächen verursacht werden, eines dem Benutzer zugeordneten Bewegungsereignisses.

2. System nach Anspruch 1, wobei das Atemtherapiesystem das Steuerungssystem, den Speicher und das Mikrofon umfasst.

3. System nach Anspruch 1, wobei es sich bei der Komponente des Atemtherapiesystems um eine Leitung des Atemtherapiesystems handelt.

4. System nach einem der Ansprüche 1 bis 3, wobei das Mikrofon außerhalb des Atemtherapiesystems angeordnet ist.

5. System nach einem der Ansprüche 1 bis 4, wobei das Mikrofon (i) innerhalb des Atemtherapiesystems angeordnet ist, (ii) in das Atemtherapiesystem integriert ist oder (iii) sowohl (i) als auch (ii) zutrifft.

6. System nach einem der Ansprüche 1 bis 5, wobei das Bewegungsereignis (i) einer Änderung der Benutzerposition, (ii) einem Schlafzustand, (iii) einer Änderung des Schlafzustands, (iv) einer Schlafphase, (v) einer Änderung der Schlafphase, (vi) einem Auftreten eines respiratorischen Ereignisses oder einer beliebigen Kombination von (i) bis (vi) zugeordnet ist.

7. System nach einem der Ansprüche 1 bis 6, wobei das Mikrofon ein erstes Mikrofon und ein zweites Mikrofon einschließt, wobei das erste Mikrofon außerhalb des Atemtherapiesystems angeordnet ist und das zweite Mikrofon (i) innerhalb des Atemtherapiesystems angeordnet ist, (ii) in das Atemtherapiesystem integriert ist oder (iii) sowohl (i) als auch (ii) zutrifft.

8. Verfahren nach einem der Ansprüche 1 bis 7, wobei das Bewegungsereignis eine Vielzahl von Bewegungsereignissen einschließt, wobei optional das Bestimmen des dem Benutzer zugeordneten Bewegungsereignisses das Bestimmen einer Bewegungshäufigkeit einschließt.

9. System nach einem der Ansprüche 6 bis 8, wobei das respiratorische Ereignis ein Einatmen, ein Ausatmen, ein Husten, ein Keuchen, ein Niesen, ein Lachen, ein Schreien, ein Kreischen, ein Schnarchen, eine Apnoe oder eine beliebige Kombination davon einschließt.

10. System (100) nach einem der Ansprüche 1 bis 9, wobei die maschinenlesbaren Anweisungen, wenn sie von dem einen oder den mehreren Prozessoren ausgeführt werden, ferner das System veranlassen zum:

Bestimmen, aus den akustischen Daten, einer Vielzahl von Audiosignalamplituden für einen bestimmten Zeitraum;
Berechnen, zumindest teilweise auf der Grundlage der bestimmten Vielzahl von Audiosignalamplituden, einer Standardabweichung für den Zeitraum; und
Vergleichen der Standardabweichung für den Zeitraum mit einem vorgegebenen Schwellenwert;

11. System nach Anspruch 10, wobei die maschinenlesbaren Anweisungen, wenn sie von dem einen oder den mehreren Prozessoren ausgeführt werden, ferner das System veranlassen zum:

Bestimmen, aus den akustischen Daten, einer Vielzahl von Basis-Audiosignalamplituden für einen ersten Zeitraum und einer Vielzahl von aktiven Audiosignalamplituden für einen zweiten Zeitraum;
Berechnen, zumindest teilweise auf der Grundlage der bestimmten Vielzahl von Basis-Audiosignalamplituden, einer Standardabweichung der Basisamplitude;
Berechnen, zumindest teilweise auf der Grundlage der bestimmten Vielzahl von aktiven Audiosignalamplituden, einer Standardabweichung der aktiven Amplitude; und
Bestimmen, zumindest teilweise auf der Grundlage der berechneten Standardabweichung der Basisamplitude und der berechneten Standardabweichung der aktiven Amplitude, des vorbestimmten Schwellenwerts;
wobei optional die Vielzahl von Basis-Audiosignalamplituden für den ersten Zeitraum keiner Bewegung oder nur geringfügiger dem Benutzer zugeordneter Bewegung entspricht, und/oder
wobei die Vielzahl von aktiven Audiosignalamplituden für den zweiten Zeitraum dem Benutzer zugeordneter aktiver Bewegungen entspricht, und/oder
wobei der vorbestimmte Schwellenwert durch Mittelung (i) der Basis-Standardabweichung und (ii) der aktiven Standardabweichung bestimmt wird.

12. System nach einem der Ansprüche 10 bis 11, wobei der Zeitraum zwei Sekunden, drei Sekunden, vier Sekunden, fünf Sekunden, sechs Sekunden, sieben Sekunden, acht Sekunden, neun Sekunden oder zehn Sekunden beträgt, und/oder wobei der Zeitraum einer Anzahl von Atemzyklen entspricht, wobei jeder Atemzyklus einen Einatmungsabschnitt und einen Ausatmungsabschnitt einschließt, wobei optional die Anzahl der Atemzyklen ein Atemzyklus, zwei Atemzyklen, drei Atemzyklen, vier Atemzyklen oder fünf Atemzyklen beträgt.

13. System nach einem der Ansprüche 1 bis 12, wobei das Analysieren der akustischen Daten eine Cepstrum-Analyse, eine Autocepstrum-Analyse, eine Autokorrelationsanalyse, eine Spektralanalyse oder eine beliebige Kombination davon einschließt, wobei optional die Spektralanalyse eine schnelle Fourier-Transformation (FFT), ein Spektrogramm, eine Kurzzeit-Fourier-Transformation (STFT), eine Wavelet-basierte Analyse oder eine beliebige Kombination davon einschließt.

14. System nach einem der Ansprüche 1 bis 13, wobei das Analysieren der akustischen Daten die Verarbeitung der empfangenen akustischen Daten zur Identifizierung einer Vielzahl von Merkmalen umfasst, die Bewegungsereignissen des Benutzers zugeordnet sind, wobei optional die Vielzahl von Merkmale (i) eine Änderung der spektralen Signatur, (ii) eine Änderung der Frequenz, (iii) eine Änderung der Signalamplitude oder (iv) eine beliebige Kombination davon einschließt.

15. System nach Anspruch 6, wobei das Mikrofon mit einer Komponente des Atemtherapiesystems gekoppelt ist, wobei die Komponente des Atemtherapiesystems aus (i) einer Leitung einer Atemtherapievorrichtung des Atemtherapiesystems, (ii) einer Leiterplatte der Atemtherapievorrichtung des Atemtherapiesystems, (iii) einem Anschluss des Atemtherapiesystems, (iv) einer Benutzerschnittstelle des Atemtherapiesystems oder (v) einer anderen Komponente des Atemtherapiesystems ausgewählt ist.

**Revendications**

1. Système (100) permettant de déterminer les mouvements d'un utilisateur lors de l'utilisation d'un système de thérapie respiratoire, ce système comprenant :

   le système de thérapie respiratoire incluant un conduit (126), une interface utilisateur (124) et un dispositif de thérapie respiratoire ;
   un microphone (140) positionné dans le conduit, l'interface utilisateur ou le dispositif de thérapie respiratoire du système de thérapie respiratoire et configuré pour générer des données acoustiques ;
   un système de commande (110) incluant un ou plusieurs processeurs (112) ; et
   une mémoire (114) sur laquelle sont stockées des instructions lisibles par machine, dans lequel les instructions lisibles par machine, lorsqu'elles sont exécutées par un ou plusieurs processeurs, amènent le système à :

   recevoir des données acoustiques associées à l'utilisateur du système de thérapie respiratoire qui sont générées via le microphone, dans lequel les données acoustiques associées à l'utilisateur sont reproductibles sous forme d'un ou plusieurs sons associés au mouvement d'un composant du système de thérapie respiratoire, dans lequel les un ou plusieurs sons associés au mouvement du composant du système de thérapie respiratoire sont causés par le frottement du composant du système de thérapie respiratoire sur une ou plusieurs surfaces ;
   analyser les données acoustiques ; et
   déterminer, sur la base des données acoustiques associées à l'utilisateur et reproductibles sous forme d'un ou plusieurs sons associés au mouvement d'un composant du système de thérapie respiratoire causé par le frottement de ce composant sur une ou plusieurs surfaces, un événement de mouvement associé à l'utilisateur.

2. Système selon la revendication 1, dans lequel le système de thérapie respiratoire comprend le système de commande, la mémoire et le microphone.

3. Système selon la revendication 1, dans lequel le composant du système de thérapie respiratoire est un conduit du système de thérapie respiratoire.

4. Système selon l'une quelconque des revendications 1 à 3, dans lequel le microphone est externe au système de thérapie respiratoire.

5. Système selon l'une quelconque des revendications 1 à 4, dans lequel le microphone est (i) interne au système de thérapie respiratoire, (ii) intégré au système de thérapie respiratoire, ou (iii) à la fois (i) et (ii).

6. Système selon l'une quelconque des revendications 1 à 5, dans lequel l'événement de mouvement est associé à (i) un changement de position de l'utilisateur, (ii) un état de sommeil, (iii) un changement d'état de sommeil, (iv) un stade de sommeil, (v) un changement de stade de sommeil, (vi) la survenue d'un événement respiratoire, ou toute combinaison de (i) à (vi).

7. Système selon l'une quelconque des revendications 1 à 6, dans lequel le microphone inclut un premier microphone et un deuxième microphone, le premier microphone étant externe au système de thérapie respiratoire, et le deuxième microphone étant (i) interne au système de thérapie respiratoire, (ii) intégré au système de thérapie respiratoire, ou (iii) à la fois (i) et (ii).

8. Procédé selon l'une quelconque des revendications 1 à 7, dans lequel l'événement de mouvement inclut une pluralité d'événements de mouvement, dans lequel, en option, la détermination de l'événement de mouvement associé à l'utilisateur inclut la détermination d'une fréquence de mouvement.

9. Système selon l'une quelconque des revendications 6 à 8, dans lequel l'événement respiratoire inclut une inhalation, une expiration, une toux, un halètement, un éternuement, un rire, un pleur, un hurlement, un ronflement, une apnée ou toute combinaison de ceux-ci.

10. Système selon l'une quelconque des revendications 1 à 9, dans lequel les instructions lisibles par la machine, lorsqu'elles sont exécutées par le un ou plusieurs processeurs, amènent en outre le système à :

déterminer, à partir des données acoustiques, une pluralité d'amplitudes de signaux audio pour une période donnée ;

en se basant au moins en partie sur la pluralité déterminée des amplitudes des signaux audio, calculer un écart type pour la période considérée ; et

comparer l'écart type de la période à un seuil prédéterminé ;

11. Système selon la revendication 10, dans lequel les instructions lisibles par machine, lorsqu'elles sont exécutées par un ou plusieurs processeurs, amènent en outre le système à :

déterminer, à partir des données acoustiques, une pluralité d'amplitudes de signaux audio de base pour une première période et une pluralité d'amplitudes de signaux audio actifs pour une deuxième période ;

en se basant au moins en partie sur la pluralité déterminée des amplitudes du signal audio de base, calculer un écart type d'amplitude de base ;

en se basant au moins en partie sur la pluralité déterminée des amplitudes des signaux audio actifs, calculer un écart type d'amplitude active ; et

en se basant au moins en partie sur l'écart type de l'amplitude de base calculée et sur l'écart type de l'amplitude active calculée, déterminer le seuil prédéterminé ;

dans lequel, éventuellement, la pluralité des amplitudes du signal audio de base pour la première période de temps correspond à une absence de mouvement ou à un mouvement mineur associé à l'utilisateur, et/ou

dans lequel la pluralité des amplitudes des signaux audio actifs pour la deuxième période correspond à un mouvement actif associé à l'utilisateur, et/ou

dans lequel le seuil prédéterminé est déterminé en faisant la moyenne (i) de l'écart type de base et (ii) de l'écart type actif.

12. Système selon l'une quelconque des revendications 10 à 11, dans lequel la période de temps est de deux secondes, trois secondes, quatre secondes, cinq secondes, six secondes, sept secondes, huit secondes, neuf secondes ou dix secondes, et/ou dans lequel la période de temps correspond à un certain nombre de cycles respiratoires, dans lequel chaque cycle respiratoire inclut une partie d'inspiration et une partie d'expiration, dans lequel le nombre de cycles respiratoires est, au choix, un, deux, trois, quatre ou cinq cycles respiratoires.

13. Système selon l'une quelconque des revendications 1 à 12, dans lequel l'analyse des données acoustiques inclut une analyse cepstrale, une analyse autocepstrale, une analyse d'autocorrélation, une analyse spectrale ou toute combinaison de celles-ci, dans lequel, en option, l'analyse spectrale inclut une transformée de Fourier rapide (FFT), un spectrogramme, une transformée de Fourier à court terme (STFT), une analyse basée sur les ondelettes ou toute combinaison de celles-ci.

14. Système selon l'une quelconque des revendications 1 à 13, dans lequel l'analyse des données acoustiques inclut le traitement des données acoustiques reçues pour identifier une pluralité de caractéristiques associées aux événements de mouvement de l'utilisateur, dans lequel, en option, la pluralité de caractéristiques inclut (i) un changement de signature spectrale, (ii) un changement de fréquence, (iii) un changement d'amplitude du signal, ou (iv) toute combinaison de ceux-ci.

15. Système selon la revendication 6, dans lequel le microphone est couplé à un composant du système de thérapie respiratoire, le composant du système de thérapie respiratoire étant choisi parmi (i) un conduit d'un dispositif de thérapie respiratoire du système de thérapie respiratoire, (ii) une carte de circuit imprimé du dispositif de thérapie respiratoire du système de thérapie respiratoire, (iii) un connecteur du système de thérapie respiratoire, (iv) une interface utilisateur du système de thérapie respiratoire, ou (v) tout autre composant du système de thérapie respiratoire.

**FIG. 1**

| | | | Sensor(s) | |
|---|---|---|---|---|
| **Control System** 110 | | 120 Respiratory System 132 | Pressure Sensor | Flow Rate Sensor 134 |
| 112 Processor | | 122 Respiratory Device | 136 Temperature Sensor | Motion Sensor 138 |
| | | 141 | Acoustic Sensor | |
| 114 Memory Device | | 124 User Interface | 140 Microphone | Speaker 142 |
| | | 147 | RF Sensor | |
| 119 Electronic Interface | | 146 126 Conduit | RF Receiver | RF Transmitter 148 |
| | | 150 | Camera | Infrared Sensor 152 |
| 170 External Device | | 128 Display Device | 154 PPG Sensor | ECG Sensor 156 |
| | | 158 EEG Sensor | Capacitive Sensor 160 |
| 172 Display Device | | 129 Humidification tank | 162 Force Sensor | Strain Gauge Sensor 164 |
| | | | 166 EMG Sensor | Oxygen Sensor 168 |
| | | | 174 Analyte Sensor | Moisture Sensor 176 |
| | | | 178 LiDAR Sensor | |

100

130

FIG. 2

FIG. 3

FIG. 4A

Pressure

FIG. 4B

401

402

403

404

405

406

407

420

Time

FIG. 4C

**FIG. 5**

500

510 — Receive acoustic data associated with the user

520 — Analyze the received acoustic data

550 — Determine a movement event associated with the user

522 — Determine a plurality of audio signal amplitudes for a time period

524 — Calculate a standard deviation for the time period

526 — Compare the standard deviation for the time period to a predetermined threshold

530 — Determine a plurality of baseline audio signal amplitudes for a first time period and a plurality of active audio signal amplitudes for a second time period

532 — Calculate a baseline standard deviation

534 — Calculate an active standard deviation

536 — Determine the predetermined threshold

540 — Process the received acoustic data to identify a plurality of features

552 — Determine a movement frequency

FIG. 6A

RF Raw - Roll_back_to_centre

FIG. 6B

Audio

FIG. 6C

Processed Audio

FIG. 6D

Movement

RF
Processed Audio

EP 4 188 192 B1

**FIG. 7A**

Activity (EmFit)

**FIG. 7B**

Activity (Audio)

**FIG. 7C**

Activity (EmFit)

**FIG. 7D**

Activity (Audio)

FIG. 8

800

810 — Receive current flow data associated with a current respiratory therapy session

820 — Analyze the current flow data to determine one or more features

832 — Compare the determined one or more features to a plurality of historical features associated with a plurality of historical body positions

830 — Determine the body position of the user

870 — Receive acoustic data

860 — Label the body position of the user with a time stamp

862 — Modify one or more parameters of the respiratory therapy session based at least in part on the determined body position for the user

864 — Generate an alarm, a notification, or an instruction based at least in part on the determined body position for the user

840 — Receive historical flow data associated with one or more prior respiratory therapy sessions

850 — Determine a plurality of historical movement events

852 — Divide each prior respiratory therapy session into a plurality of respiratory therapy sections

854 — Analyze the received historical flow data to extract a plurality of historical features

856 — Associate the plurality of historical features with one or more historical body positions

EP 4 188 192 B1

FIG. 9

FIG. 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62706104
- WO 2020092701 A2
- WO 2008138040 A
- US 9358353 B
- WO 2016061629 A
- US 20170311879 A
- WO 2014047310 A
- US 20140088373 A
- WO 2017132726 A
- WO 2019122413 A
- WO 2019122414 A
- US 20200383580 A
- WO 2012012835 A
- US 10328219 B
- WO 2018050913 A
- WO 2020104465 A
- WO 2010091462 A

**Non-patent literature cited in the description**

- **CHILDERS et al.** The Cepstrum: A Guide to Processing. *Proceedings of the IEEE*, October 1977, vol. 65 (10)
- **RANDALL RB**. Frequency Analysis. Bruel & Kjaer, 1977, 344